# EUROPEAN PATENT SPECIFICATION

(11) **EP 3 802 922 B1**
(45) Date of publication and mention of the grant of the patent: **26.04.2023**
(21) Application number: 19740098.9
(22) Date of filing: 10.06.2019
(51) Int. Cl.: C40B 30/04, G01N 33/574

(54) **NOVEL IMMUNE CHECKPOINT INHIBITORS**
NEUE IMMUNCHECK-INHIBITOREN
NOUVEAUX INHIBITEURS DE POINT DE CONTRÔLE IMMUNITAIRE

(30) Priority: 11.06.2018 US 201862683256 P
(43) Date of publication of application: 14.04.2021
(73) Proprietor: Yale University, New Haven, CT 06510 (US); Boehringer Ingelheim International GmbH, 55216 Ingelheim am Rhein (DE)
(72) Inventor: BLAIR, David A., Ridgefield, Connecticut 06877-0368 (US); CHEN, Lieping, New Haven, Connecticut 06510 (US); WANG, Jun, New Haven, Connecticut 06510 (US); ZHENG, Timothy, Ridgefield, Connecticut 06877-0368 (US)
(74) Representative: Simon, Elke Anna Maria
(86) International application number: PCT/US2019/036252
(87) International publication number: WO 2019/241098

(56) References cited:
- WO-A1-2015/042246
- WO-A1-2017/078318
- WO-A1-2019/141092
- WO-A2-2010/019570
- CN-A- 110 029 168
- CN-B- 102 393 456
- KR-A- 20150 037 321
- JUN YAN ET AL: "LFIRE-1/HFREP-1, a liver-specific gene, is frequently downregulated and has growth suppressor activity in hepatocellular carcinoma", ONCOGENE, vol. 23, no. 10, 23 February 2004 (2004-02-23), pages 1939-1949, XP055383539, London ISSN: 0950-9232, DOI: 10.1038/sj.onc.1207306
- HAMED NAYEB-HASHEMI ET AL: "Targeted disruption of fibrinogen like protein-1 accelerates hepatocellular carcinoma development", BIOCHEMICAL AND BIOPHYSICAL RESEARCH COMMUNICATIONS, vol. 465, no. 2, 18 September 2015 (2015-09-18), pages 167-173, XP055383533, AMSTERDAM, NL ISSN: 0006-291X, DOI: 10.1016/j.bbrc.2015.07.078
- JUN WANG ET AL: "Fibrinogen-like Protein 1 Is a Major Immune Inhibitory Ligand of LAG-3", CELL, vol. 176, no. 1-2, 10 January 2019 (2019-01-10), pages 334-347.e12, XP055629972, AMSTERDAM, NL ISSN: 0092-8674, DOI: 10.1016/j.cell.2018.11.010
- CREG J WORKMAN ET AL: "Phenotypic analysis of the murine CD4-related glycoprotein, CD223 (LAG-3)", EUROPEAN JOURNAL OF IMMUNOLOGY, WILEY-VCH, HOBOKEN, USA, vol. 32, no. 8, 26 July 2002 (2002-07-26), pages 2255-2263, XP071222330, ISSN: 0014-2980, DOI: 10.1002/1521-4141(200208)32:8<2255::AID-IM MU2255>3.0.CO;2-A

## Description

### TECHNICAL FIELD

The present invention relates to the identification of fibrinogen like protein 1 (FGL1) as a target for the treatment of a cancer patient and as a prognostic biomarker for predicting responsiveness of such cancer patient to an immune checkpoint inhibitor. More specifically it relates to an antibody specifically inhibiting the interaction of fibrinogen-like protein 1 (FGL1) with lymphocyte-activation gene 3 protein (LAG3), and/or an antibody specifically binding to FGL1, and their use in treating cancer in a human patient as defined in the appended claims, wherein the cancer is preferably an FGL1 expressing cancer. Further, the invention relates to methods for assessing susceptibility or predicting the responsiveness to the treatment with an immune checkpoint inhibitory antibody in a cancer patient, comprising detecting FGL1 expression in a sample from said patient. The immune checkpoint inhibitory antibody may be an antibody directed against human FGL1 or an antibody directed against human LAG3, optionally in combination with other immune checkpoint inhibitory antibodies, such as an anti-PD1 antibody or an anti-PD-L1 antibody.

### BACKGROUND

Upregulated expression of inhibitory receptors (IRs) is essential to balance co-stimulatory receptor activity and limit T-cell activation, thereby preventing autoimmunity, autoinflammation, and tissue damage. However, tumors can hijack these so-called immune checkpoint mechanisms as protection against anti-tumor immune responses elicited by CD4+ and CD8+ T cells. Recent cancer immunotherapeutic approaches have aimed to reverse such exhaustion by targeting inhibitory receptors to overcome immune tolerance allowing reactivation of cytotoxic T cell responses against tumors.

A number of immunomodulatory agents mainly antibodies that target immune system checkpoints, such as cytotoxic T-lymphocyte antigen 4 (CTLA-4) or programmed death-1 (PD-1) and its ligand (PD-L1), have received regulatory approval for the treatment of multiple cancers including malignant melanoma, non-small cell lung cancer, renal cell carcinoma, classical Hodgkin lymphoma, and recurrent or metastatic head and neck squamous cell carcinoma. However, a substantial proportion of patients treated with checkpoint inhibitors have little or no benefit and these treatments are costly and might have some associated toxicities. Thus, there is a need for further checkpoint inhibitors, for new combinations of checkpoint inhibitors and/or for prognostic biomarkers for predicting treatment responses to immune checkpoint inhibitory antibodies in cancer therapy in order to optimize patient selection.

Another immune checkpoint receptor is lymphocyte activated gene-3 (LAG3, CD223). LAG3 upregulation is required to control over-activation of the immune system and to prevent autoimmunity. It is a potential cancer immunotherapeutic target due to its negative regulatory role on T cells. LAG3 is a cell surface T-cell suppressive protein and is believed to function via interacting with major histocompatibility complex class II (MHC-II). There are now several LAG3-modulating immunotherapeutics at various stages of clinical and preclinical development, such as the LAG3-Fc fusion protein IMP321 and the anti-LAG3 antibody BMS-986016. Current anti-LAG3 therapy targets the interaction with its only known ligand, MHC class II. For example, WO2010019570 discloses isolated monoclonal antibodies that specifically bind to LAG-3 and that can inhibit the binding of LAG-3 to MHC Class II molecules. However, interaction with MHC-II does not seem to be required for LAG3-induced suppressive function in various experimental settings, thus allowing for speculations as to potential additional functional LAG3 ligands.

Fibrinogen like protein 1 (FGL1), synonyms used in the literature LFIRE-1 or HFREP-1, is a liver-enriched soluble protein with hepatocyte mitogenic activity and metabolic functions. Yan et al. show that *LFIRE-1* and *HFREP-1* are specifically expressed in normal human liver tissue, but reduced or undetectable in most of hepatocellular carcinoma (HCC) specimens at both RNA and protein level. Furthermore, Yan et al. describe that the reduction or non-expression of *LFIRE-1*/*HFREP-1* is significantly associated with the degree of tumor differentiation (Yan et al. Oncogene 2004; 23: 1393-1949).

Nayeb-Hashemi et al. describe that mice lacking FGL1 develop HCC at more than twice the rate of wild type mice. They thus hypothesize that FGL1 acts as a tumor suppressor in hepatocellular cancer (Nayeb-Hashemi et al., Biochem Biophys Res Commun. 2015; 465(2): 167-173).

### SUMMARY OF THE INVENTION

The inventors identified fibrinogen like protein 1 (FGL1), a liver-enriched soluble protein with hepatocyte mitogenic activity and metabolic functions, as a LAG3 binding partner with important implications for cancer immunotherapy. Given the high expression of FGL1 in primary resistant patients with anti-PD1/PD-L1 therapy, the FGL1/LAG3 pathway may be a further potential mechanism for immune escape. Thus, FGL1 and its interaction with LAG3 is a potential target for therapeutic agents, and expression of FGL1 is a promising prognostic biomarker for predicting response to treatment with an immune checkpoint inhibitor agent, such as an antibody that inhibits the interaction between FGL1 and LAG3. Since FGL1/LAG3 checkpoint inhibition seems to complement at least PD-1/PD-L1 checkpoint inhibition, FGL1 may also be a negative prognostic marker for other immune checkpoint inhibitory antibodies such as anti-PD-1 or anti-PD-L1 antibodies.

Provided according to the invention is an antibody specifically inhibiting the interaction of fibrinogen-like protein 1 (FGL1) with lymphocyte-activation gene 3 protein (LAG3) for use in the treatment of cancer in a human patient.

Provided herein is an antibody specifically inhibiting the interaction of FGL1 with LAG3 (which is considered to be an "immune checkpoint inhibitory antibody", as it interacts with an immune checkpoint pathway) for use in treating cancer in a human patient, as characterized in the appended claims. Further provided is an antibody specifically binding to human FGL1 for use in treating cancer in a human patient, as characterized in the appended claims. In particular embodiments, the cancer patient has an elevated FGL1 expression, preferably the cancer patient has been determined to have elevated FGL1 expression. More specifically, a sample from the patient has been determined to have elevated FGL1 expression, preferably a sample obtained from the patient has been determined to have elevated FGL1 expression compared to a control. Preferably, the elevated FGL1 expression has been determined in a sample from the patient *in vitro.* The antibody for use may comprise determining FGL1 expression in a sample from the patient, before the anti-FGL1 antibody or the antibody specifically inhibiting the interaction of FGL1 with LAG3 is to be administered to the patient. In one embodiment the use comprises determining if FGL1 expression is elevated in a sample from the patient, and if FGL1 expression is elevated, the anti-FGL1 antibody or the antibody specifically inhibiting the interaction of FGL1 with LAG3 is to be administered to the patient.

Further described herein is a method of treating cancer in a human patient or the antibody for use, comprising a) providing a sample from the patient, b) measuring FGL1 expression level in said sample, c) comparing FGL1 expression to a control, d) identifying the patient as likely responsive to treatment with an anti-FGL1 antibody or an antibody specifically inhibiting the interaction of FGL1 with LAG3 when FGL1 expression level has been measured as elevated compared to the control, and e) administering an effective amount of said anti-FGL1 antibody or said antibody specifically inhibiting the interaction of FGL1 with LAG3 to the patient.

The present invention further relates to a method of selecting a therapy comprising an anti-FGL1 antibody or an antibody specifically inhibiting the interaction of FGL1 with LAG3 for a cancer patient, the method comprising determining FGL1 expression level in a sample from the patient and selecting said therapy for the patient when the FGL1 expression levels is elevated, as characterized in the appended claims.

The FGL1 expression according to the methods or the antibody for use of the invention is preferably elevated compared to a control. The control may be a reference value or a reference sample for base line expression from at least one subject not having cancer or a non-cancerous tissue sample, wherein the non-cancerous tissue sample preferably originates from the same organ as the cancer and more preferably from the cancer patient. The sample from the patient as used in the methods or the uses according to the invention is preferably a tumor sample and/or a blood sample.

The antibody specifically inhibiting the interaction of FGL1 with LAG3 used in the methods or therapies of the invention and as described herein may be an anti-FGL1 antibody, an anti-LAG3 antibody, or combinations thereof. The immune checkpoint inhibitory antibody specifically inhibiting the interaction of FGL1 with LAG3 may be used alone or in combination with at least one further immune checkpoint inhibitory antibody selected from the group consisting of an anti-PD-1 antibody, an anti-PD-L1 (anti-B7-H1) antibody, an anti-IDO antibody, an anti-CTLA-4 antibody, an anti-Tim-3 antibody, an anti-GITR antibody, an anti-VISTA antibody, an anti-BTLA antibody, and an anti-OX40 antibody. In particular embodiments the at least one further immune checkpoint inhibitory antibody is an anti-PD-1 antibody or an anti-PD-L1 antibody. In particular embodiments the patient may have a primary or acquired resistance to at least one checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, and an anti-OX40 antibody. More specifically, the patient may have a primary or acquired resistance to PD-1/PD-L1 checkpoint inhibition.

The cancer as referred to above may be without being limited thereto, a brain cancer, a colorectal cancer, a melanoma, a prostate cancer or a lung cancer. Preferably the cancer is a lung cancer or a prostate cancer. More preferably, the lung cancer is a non-small cell lung cancer (NSCLC). The treatment may be further accompanied by chemotherapy or radiotherapy.

In another aspect a method of detecting elevated FGL1 expression in a human cancer patient is described, said method comprises providing a sample from the patient; and detecting FGL1 expression in the sample, wherein the detected FGL1 expression in the sample is preferably compared to a control. The method may optionally further comprise identifying the patient as likely susceptible to the treatment with an anti-FGL1 antibody or an immune checkpoint inhibitory antibody specifically inhibiting the interaction of FGL1 with LAG3 when the FGL1 expression has been determined as being elevated compared to the control.

Also described herein is a method of assessing susceptibility to the treatment with an anti-FGL1 antibody or an antibody specifically inhibiting the interaction of FGL1 with LAG3 in a cancer patient, comprising a) optionally providing a sample from the patient, b) detecting FGL1 expression in the sample, c) comparing the FGL1 expression determined in step (b) to a control, wherein the FGL1 expression in the sample is elevated compared to a control and optionally further comprising identifying the patient as likely susceptible to the treatment with an anti-FGL1 antibody or an antibody specifically inhibiting the interaction of FGL1 with LAG3 when the FGL1 expression has been determined as being elevated compared to the control.

Also described is a method of categorizing a tumor of a patient, comprising (a) optionally providing a sample from the patient, (b) detecting FGL1 expression in the sample, and (c) identifying the tumor as a candidate for the treatment with an anti-FGL1 antibody or an immune checkpoint inhibitory antibody, preferably an antibody specifically inhibiting the interaction of FGL1 with LAG3. Optionally the method further comprises a step of comparing the FGL1 expression determined in step (b) to a control, wherein an elevated FGL1 expression in the sample compared to a control identifies the tumor as a candidate for the treatment with an anti-FGL1 antibody or an immune checkpoint inhibitory antibody, preferably an antibody specifically inhibiting the interaction of FGL1 with LAG3. Specifically, provided herein is an *in vitro* method of categorizing a tumor of a patient and/or assessing susceptibility of a cancer patient to the treatment with an immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, an anti-OX40 antibody, an anti-FGL1 antibody, an anti-LAG3 antibody and preferably an antibody that specifically inhibits the interaction of FGL1 with LAG3, comprising
(a) providing a sample from the patient,
(b) detecting FGL1 expression in the sample, and
(c) comparing said FGL1 expression determined in step (b) to a control, wherein said FGL1 expression in said sample is elevated compared to said control, wherein the control is a reference value or a reference sample for base line expression from at least one subject not having cancer or a non-cancerous tissue sample, wherein the non-cancerous tissue sample preferably originates from the same organ as the cancer and more preferably from the cancer patient.

Also described herein is a method of predicting the responsiveness of a human cancer patient comprising (a) optionally providing a sample from the patient, (b) determining FGL1 expression level in said sample, (c) comparing FGL1 expression to a control, and (d) identifying the patient as likely responsive to treatment with an anti-FGL1 antibody or with an antibody specifically inhibiting the interaction of FGL1 with LAG3, when FGL1 expression level has been measured as elevated compared to the control, or identifying the patient as less likely responsive when FGL1 expression has not been measured elevated compared to the control. Specifically, provided herein is a method of predicting the responsiveness of a human cancer patient, comprising
(a) providing a sample from the patient,
(b) determining FGL1 expression level in said sample,
(c) comparing FGL1 expression to a control,
(d) identifying the patient as likely responsive to treatment with an immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, an anti-OX40 antibody, an anti-FGL1 antibody, an anti-LAG3 antibody and preferably an antibody that specifically inhibits the interaction of FGL1 with LAG3, when FGL1 expression level has been determined as elevated compared to the control, or identifying the patient as less likely responsive when FGL1 expression has been determined as not elevated compared to the control, wherein the control is a reference value or a reference sample for base line expression from at least one subject not having cancer or a non-cancerous tissue sample, wherein the non-cancerous tissue sample preferably originates from the same organ as the cancer and more preferably from the cancer patient.

Also described is a method of predicting the responsiveness of a human cancer patient comprising (a) optionally providing a sample from the patient treated or previously treated with at least one immune checkpoint inhibitory antibody, (b) determining FGL1 expression level in said sample, (c) comparing FGL1 expression to a control, and (d) identifying the patient as less likely responsive to treatment with said at least one immune checkpoint inhibitory antibody when FGL1 expression level has been determined as elevated compared to the control.

The method of detecting elevated FGL1 expression, the method of assessing susceptibility, the method of categorizing and the method of predicting the responsiveness described herein are typically and preferably *in vitro* methods. In particular embodiments the patient from which the sample is provided may have a primary or acquired resistance to at least one checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, and an anti-OX40 antibody. More specifically, the patient may have a primary or acquired resistance to PD-1/PD-L1 checkpoint inhibition. These methods may further comprise or be followed by a step of administering to the cancer patient an anti-FGL1 antibody or an antibody specifically inhibiting the interaction of FGL1 with LAG3. The sample used in any one of the methods is preferably a tumor or a blood sample. The FGL1 expression may be detected in the sample (a) by contacting the sample with an anti-FGL1 antibody and detecting the binding between FGL1 (FGL1 protein) and the antibody, or (b) detecting the amount of mRNA encoding FGL1 in the sample. In particular embodiments the control is a reference value or a reference sample for base line expression from at least one subject not having cancer or a non-cancerous tissue sample, wherein the non-cancerous tissue sample preferably originates from the same organ as the cancer and more preferably from the cancer patient. In particular embodiment the immune checkpoint inhibitory antibody that inhibits the interaction of FGL1 with LAG3, is an anti-LAG3 antibody or an anti-FGL1 antibody.

Also described herein is a method for treating cancer in a human patient comprising administering to the patient an effective amount of an anti-FGL1 antibody or of an antibody specifically inhibiting the interaction of FGL1 with LAG3, wherein the cancer patient has an elevated FGL1 expression determined using any one of the diagnostic methods described herein.

Also described herein is a kit for selecting a cancer patient that would benefit from an immune checkpoint inhibitory antibody, preferably an anti-FGL1 antibody or an antibody specifically inhibiting the interaction of FGL1 with LAG3, wherein the kit comprises means for quantitatively detecting FGL1 expression in a sample from a cancer patient, and a control. The control may be selected from the group consisting of (a) a reference sample for detecting enhanced FGL1 expression, (b) a reference sample for detecting base line FGL1 expression, (c) instructions containing a predetermined reference level of FGL1 expression that has been correlated with susceptibility to an immune checkpoint inhibitory antibody, preferably an anti-FGL1 antibody or an antibody specifically inhibiting the interaction of FGL1 with LAG3, (d) instructions containing a predetermined reference level of FGL1 expression that has been correlated with not being susceptible to an immune checkpoint inhibitory antibody, preferably an anti-FGL1 antibody or an antibody specifically inhibiting the interaction of FGL1 with LAG3, and/or combinations of (a), (b), (c) or (d). The means for quantitatively detecting FGL1 expression in a sample from a cancer patient may be (a) an antibody specific for FGL1, (b) a primer pair specific for FGL1 and optionally a probe hybridizing to the amplified product, and/or (c) a probe hybridizing to the FGL1 sequence.

In yet another aspect a method for screening for an immune checkpoint inhibitory antibody is provided as defined by the appended claims, comprising
(a) providing a FGL1 protein, preferably comprising at least the fibrinogen domain of human FGL1;
(b) providing a LAG3 protein, preferably comprising at least domains 1 and 2 of human LAG3;
(c) contacting the FGL1 protein and the LAG3 protein in the presence of an antibody to be screened;
(d) determining binding of the FGL1 protein and the LAG3 protein; and
(e) identifying the antibody to be screened as an immune checkpoint inhibitory antibody that specifically inhibits interaction of the FGL1 protein with the LAG3 protein if the binding of the FGL1 protein to the LAG3 protein is reduced compared to the binding of the FGL1 protein to the LAG3 protein in the absence of said antibody to be screened. Preferably, the antibody is a human or humanized antibody.

In yet another aspect a method for screening for a small molecule inhibiting the binding of (human) FGL1 to (human) LAG3 is provided as defined in the appended claims, comprising
(a) providing a FGL1 protein, preferably comprising at least the fibrinogen domain of human FGL1;
(b) providing a LAG3 protein, preferably comprising at least domains 1 and 2 of human LAG3;
(c) contacting the FGL1 protein and the LAG3 protein in the presence of a small molecule, e.g. from a compound library, e.g. in the format of a high throughput screen,
(d) determining binding of the FGL1 protein to the LAG3 protein in the presence and in the absence of said small molecule; and
(e) identifying a small molecule that specifically inhibits interaction of said FGL1 protein with said LAG3 protein as a FGL 1-LAG3 checkpoint inhibitor molecule, suitable for developing a drug for the treatment of cancer diseases as mentioned hereinbefore.

Provided herein is an immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, an anti-OX40 antibody, an anti-FGL1 antibody, an anti-LAG3 antibody and preferably an antibody that specifically inhibits the interaction of FGL1 with LAG3, for use in the treatment of cancer in a human patient, wherein the cancer patient has an elevated FGL1 expression compared to a reference value or a reference sample for base line expression from at least one subject not having cancer or a non-cancerous tissue sample, wherein the non-cancerous tissue sample preferably originates from the same organ as the cancer and more preferably from the cancer patient.

Also provided herein is an antibody that specifically binds to human FGL1, wherein the antibody inhibits binding of human FGL1 to human LAG3.

Further provided herein is an antibody that specifically binds to human LAG3, wherein the antibody inhibits binding of human LAG3 to human FGL1, wherein the antibody further inhibits interaction of human LAG3 with human MHC class II molecule.

According to a specific embodiment, an anti-FGL1 antibody for use in the treatment of cancer in a human patient is provided herein, wherein the cancer patient has an elevated FGL1 expression compared to a reference value or a reference sample for base line expression from at least one subject not having cancer or a non-cancerous tissue sample, wherein the non-cancerous tissue sample preferably originates from the same organ as the cancer and more preferably from the cancer patient.

### DESCRIPTION OF THE FIGURES

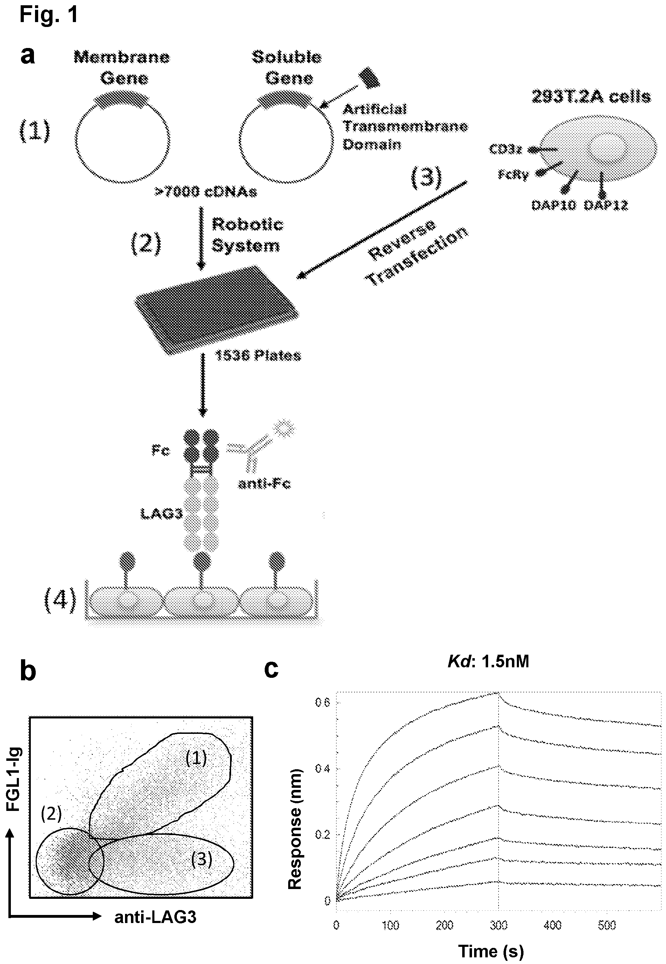

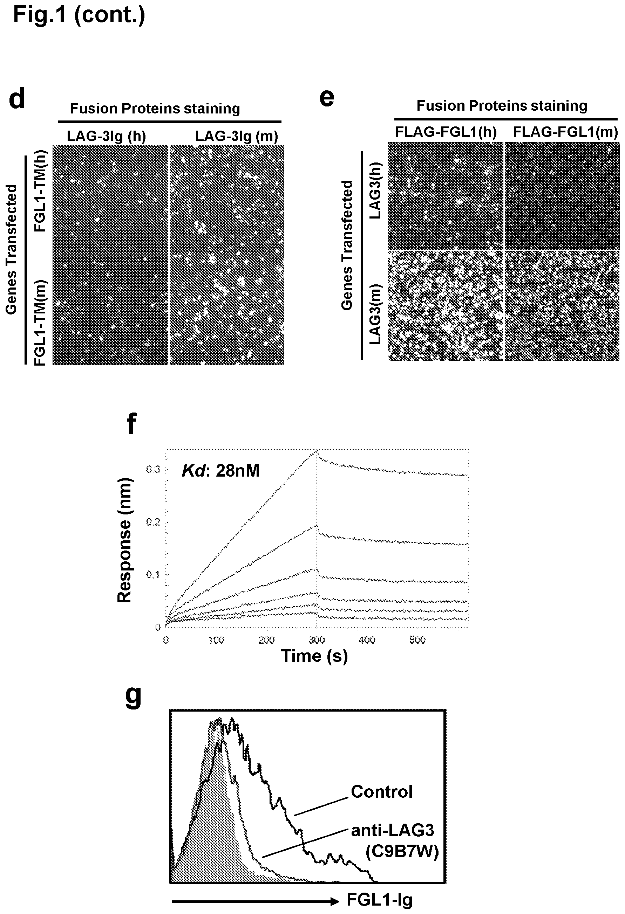

**Figure 1****. Identification of FGL1 as a novel LAG3 binding partner**
   (a) Schematic representation of the Genome Scale Receptor Array screening in the following steps: (1) Collection of cDNA library encoding both plasma membrane genes and soluble genes. Selected soluble genes were fused with artificial transmembrane domain to facilitate the membrane expression and array detection. (2) Robotic-based receptor array preparation in 1536-well plates. (3) Reverse transfection of gene library in the receptor array by an ultra-speed orbital shaker based procedure. 293T.2A, an engineered 293T cell line over-expressing several immune-associated adaptors was used for this assay. (4) Receptor Array screening for LAG3-Ig, the collection and analysis of the array results. Human Fc Receptors served as internal positive controls within each plate for screened fusion proteins. (b) Representative flow cytometry dot plot of FGL1-lg binding to mouse LAG3-stably infected 293T.2A (1) or Mock cells (2). Control Ig binding to mouse LAG3 expressing cells are indicated (3). All samples were stained with anti-LAG3. (c) Representative Octet sensorgrams of mouse FGL1 binding to immobilized mouse LAG3 fusion protein. (d-e) Interactions between FGL1 and LAG3. 293T.2A cells were transfected with full-length human or mouse FGL1-TM (FIBCD1(1-57) fused to FGL1(23-312)) (d) or LAG3 (e) as indicated on the *y*-axis. Fusion proteins were added to the culture as indicated on the x-axis to evaluate binding to the transfectants by cellular detection system (CDS). Screenshots of individual wells captured by CDS analysis software were shown. (f) Octet analysis of human FGL1-LAG3 interaction. The Octet Protein A sensor was loaded with human LAG3-Fc and then tested for binding kinetics with human FLAG-FGL1 as indicated. The Kd value was calculated by Octet software using 1:1 binding ratio. (g) FGL1-lg binding to mouse LAG3 transfected 293T.2A cells, in the presence of control antibodies (control) or anti-LAG3 (C9B7W), was tested by FACS. Control Fc fusion protein binding was served as a negative control (shadow line).

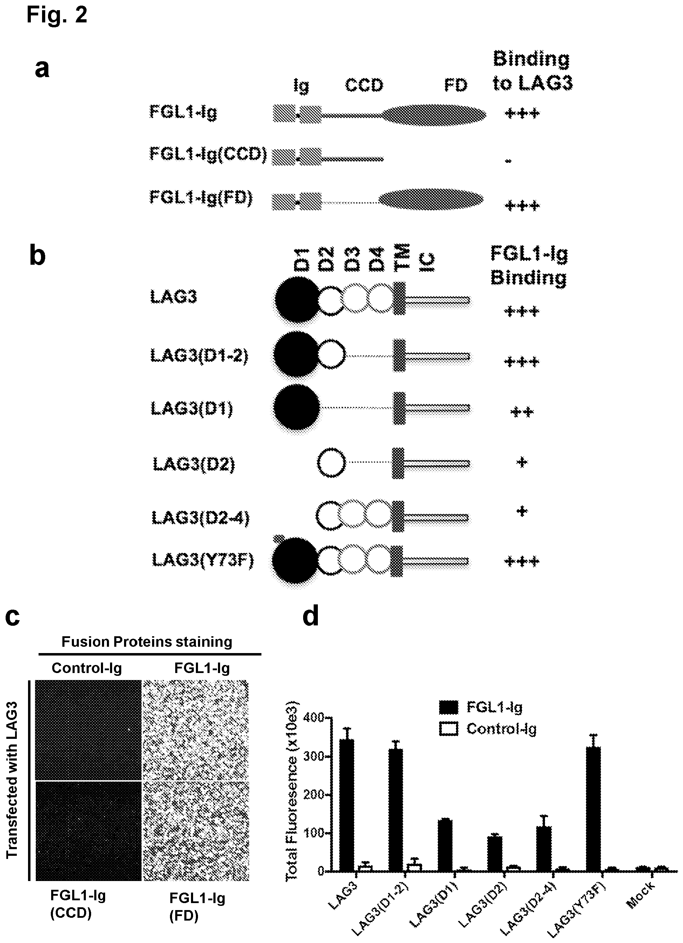

**Figure 2****. Characterization of FGL1-LAG3 interaction**
   (a) The binding of mouse FGL1-lg, FGL1-lg with coiled-coil domain (CCD), or with fibrinogen domain (FD) to mouse LAG3 transfected 293T.2A cells was measured by cellular detection system, (b) FGL1-Ig binding to 293T.2A cells transfected with full-length mouse LAG3, or LAG3 with different extracellular domain deletions. LAG3 full-length protein consists of 4 extracellular Ig domains (D1 to D4), the transmembrane domain (TM), and intracellular domain (IC). Binding intensity was presented as "+++" (strong), "++" (medium), "+" (weak), and "-" (no binding). (c) 293T.2A cells were transfected with full-length mouse LAG3. FGL1-lg with different domain deletions or control Fc Fusion proteins were added to the culture as indicated and their binding to the transfectants was measured by CDS. (d) Quantification of FGL1 binding to LAG3 domain deletion/mutation forms as in (c), by CDS software.

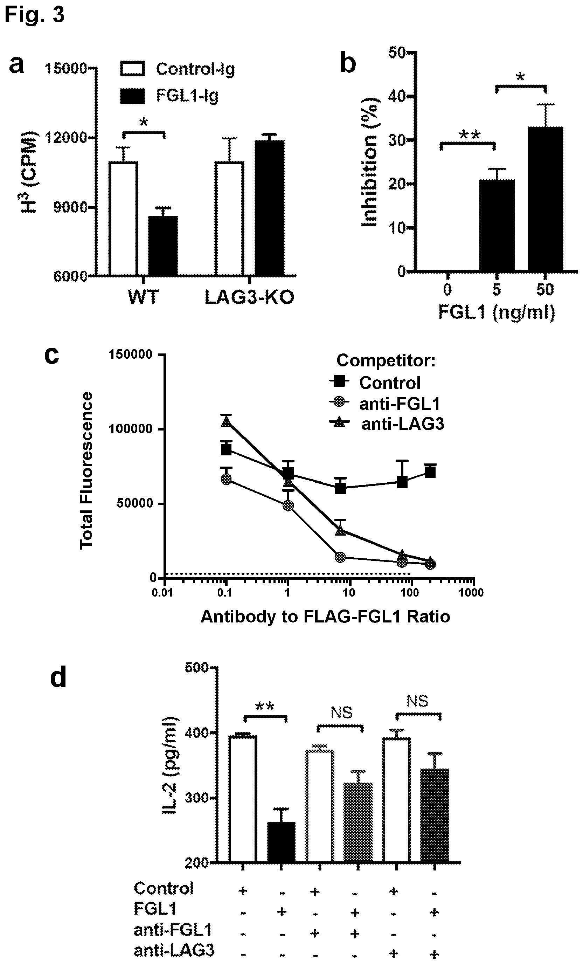

**Figure 3****. FGL1 shows LAG3 dependent inhibitory activities on T cells**
   (a) Splenic T cells from WT or LAG3-KO mice were activated by immobilized anti-CD3 antibodies at suboptimal concentration in the presence of soluble FGL1-lg or control-lg (5ug/ml) for 3 days before the addition of ³H-dTR. The thymidine incorporation of proliferated T cells was analyzed 16hrs later. (b) The 3A9-LAG3 mouse T cell hybridoma cells were co-cultured with LK35.2 B cell line in *CellGro* serum free medium, in the presence of HEL peptide, and different concentrations of recombinant FGL1 (ranging from 0, 5, 50 ng/ml). Shown is the normalized percentage of inhibition on the IL-2 levels in the supernatant at 24hrs (no FGL1 as 0% inhibition). (c) Binding of mouse FLAG-FGL1 (100ng/ml) to mouse LAG3 transfected 293T.2A cells in the presence of anti-FGL1, anti-LAG3, or control antibodies at varying antibody: FGL1 ratios was quantified by CDS. (d) 3A9-LAG3 mouse T cell hybridoma cells were co-cultured with LK35.2 B cell line as in (c), in the presence of HEL peptide and recombinant FGL1(50ng/ml), anti-FGL1, and anti-LAG3 antibodies (1ug/ml). Shown are the IL-2 levels in the supernatant at 24hrs. *, p<0.05; **, p<0.01; NS, not significant.

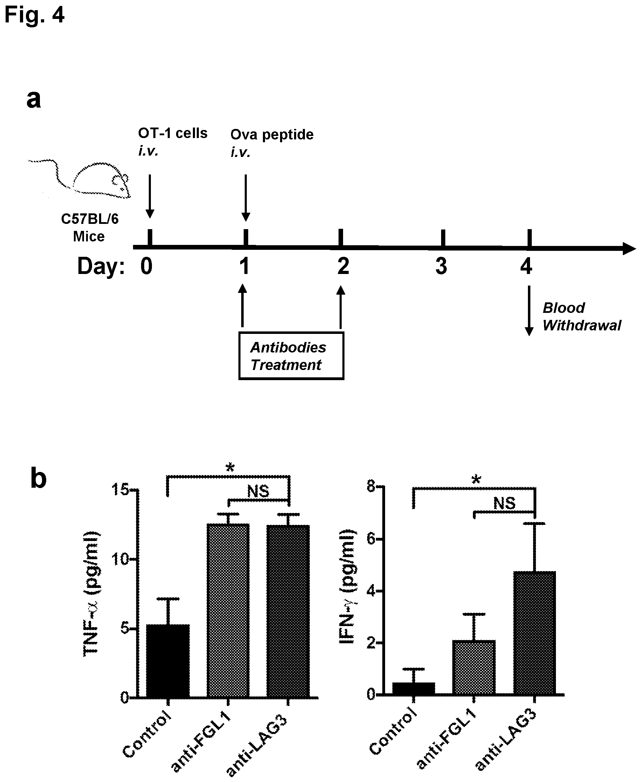

**Figure 4****. Anti-FGL1 potentiates antigen specific T cell responses *in vivo***
   (a) Schematic representation of the experimental design. 2x10e6 splenocytes from OT-1/Rag2KO mice were transferred *i.v.* into C57/BL6 mice, followed by the treatment with the ova peptide (500ug per mouse) one day later. Injections of control, anti-FGL1, or anti-LAG3 antibodies were performed at day 1 and day 2. (b) Plasma cytokine levels at day 4 were measured by CBA. *, p<0.05; NS, not significant.
**Figure 5****. FGL1-KO mice impart a pro-inflammatory phenotype**
   (a-b) The FGL1 expression value in different mouse tissues and immune cell subsets as indicated in BioGPS microarray database.
**Figure 6****. Enhanced immune responses in the blood of FGL1-KO mice**
   (a) Frequency of immune lineages indicated of CD45 compartment from peripheral blood of WT and FGL1 KO mice. (b) Quantification of indicated immune subsets in peripheral blood of WT (filled) and FGL1 KO (striped) mice. *, p<0.05
**Figure 7****. FGL1 deficiency or blockade delays tumor growth**
   (a-b) FGL1-KO, LAG3-KO, or WT littermates (WT) were inoculated with MC38 tumor cells (0.5x10e6 per mouse) at day 0. Mean tumor diameter (a) and survival (b) of these mice at indicated days were monitored after tumor inoculation. (c-f) C57/BL6 mice were inoculated with MC38 tumor cells (0.5x10e6 per mouse) at day 0 and treated with anti-FGL1, anti-LAG3, or control mAbs every four days from day 6 to day 18. Tumor response (c) and the absolute number of CD45 (left panel), CD8 (middle panel) and CD4 (right panel) cells per mg of tumor (d) are depicted. Also, graphs bar (e) showed the functional phenotype of CD8 TILs in tumors from FGL-1 KO and anti-FGL1 treated mice. (f) WT or FGL1-KO mice were inoculated with MC38 tumor cells and treated with anti-LAG3 or control antibodies as in (c). Mean tumor diameter of these mice was shown. (g) LAG3-KO mice were inoculated with MC38 tumor cells and treated with anti-FGL1 or control antibodies as in (c). Mean tumor diameter of these mice was shown. *, p<0.05; **, p<0.01; ***, p<0.005, ****, p<0.001, NS, not significant.

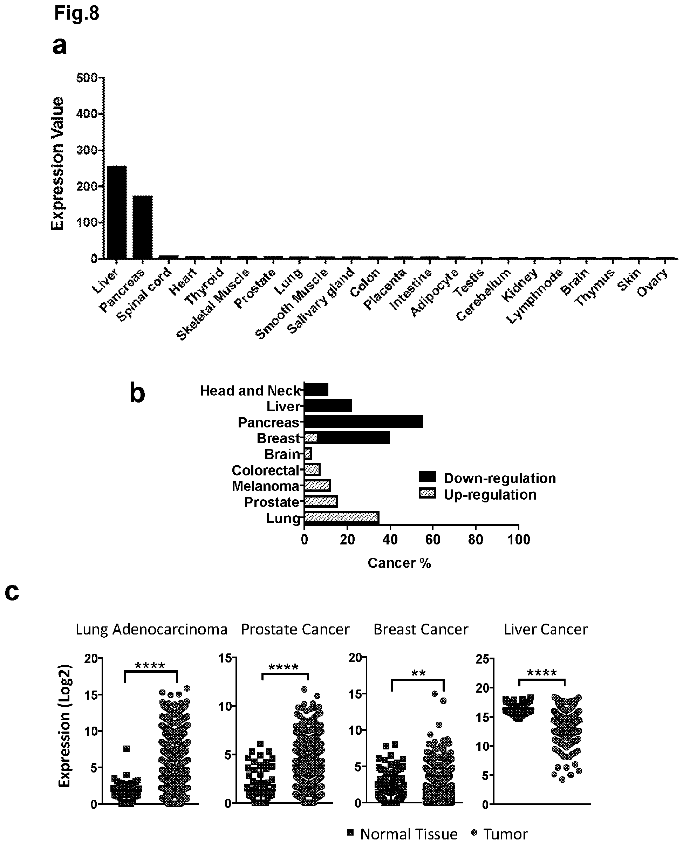

**Figure 8****. FGL1 expression is upregulated in solid cancers**
   (a) FGL1 expression value in different human tissues as indicated in BioGPS microarray database (mRNA). (b) Frequency of studies with FGL1 gene upregulation or downregulation (P value <0.05, fold change>3 or <0.3 as cutoff) in cancer lesions compared to the counterpart normal tissues in major solid cancers were analyzed by R program based on the Oncomine databases. (c) FGL1 expression value (mRNA) lung adenocarcinoma and other solid cancers (left hand side) compared to the counterpart normal tissues (right hand side) was analyzed by using TCGA cancer databases.

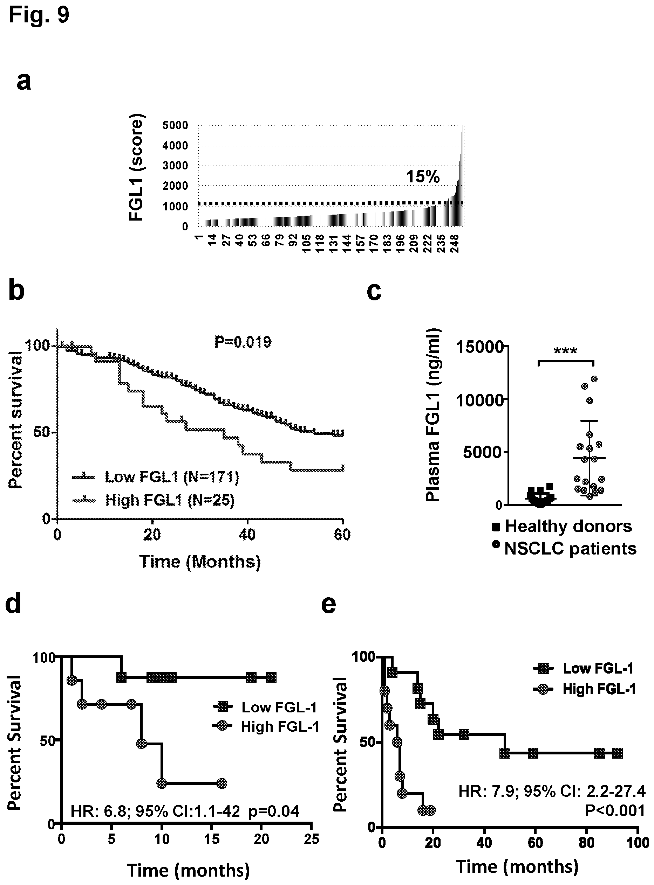

**Figure 9****. FGL1 is upregulated in human cancers and has prognostic value**
   (a) FGL1 expression distribution as indicated by quantitative immunofluorescence (QIF) staining in YTMA250 NSCLC cancer tissue arrays staining for FGL1, DAPI nuclear and cytokeratin. The median expression value from normal lung tissues was used as a cutoff for FGL1 upregulation. (b) Association of tumor FGL1 expression quantified by quantitative immunofluorescence with overall patient survival in YTMA250 lung cancers. (c) Plasma FGL1 levels in a Spain cohort of NSCLC cancer patients and healthy donors were tested by ELISA. (d-e) Survival analysis of FGL1 high or FGL1 low NSCLC (d) or melanoma (he patients after anti-PD1 therapy.

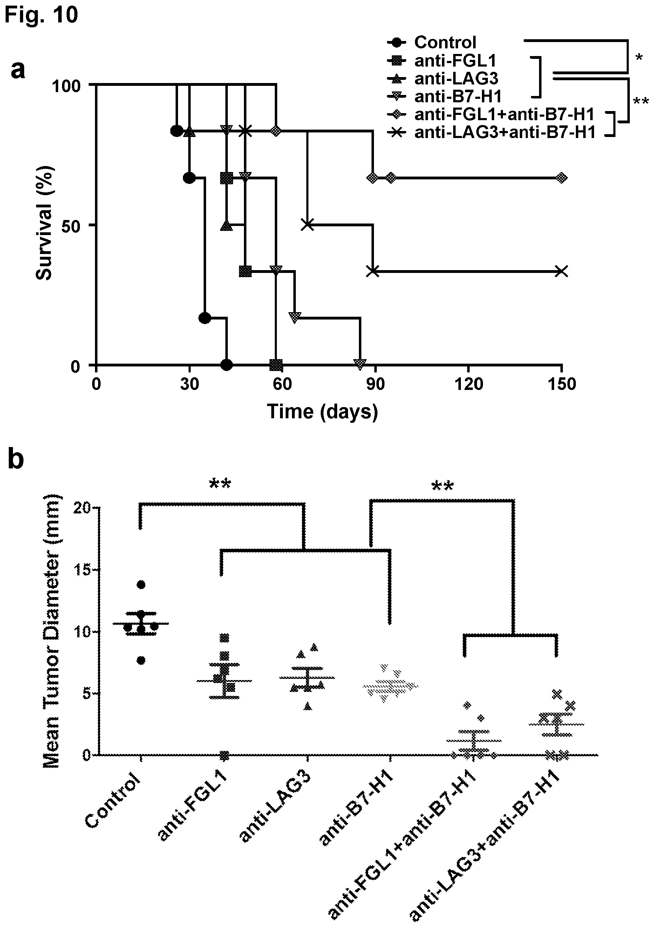

**Figure 10****. Synergistic effect of FGL1 targeting with anti-PD therapy**
   (a) C57/BL6 mice were inoculated with MC38 tumor cells (0.5x10e6 per mouse) at day 0, followed by treatment with anti-FGL1, anti-LAG3, or control antibodies every four days from day 6 to day 18. In some groups, mice were also treated with one single dose of anti-B7-H1 (10B5) at day 6. The survival of the mice in these groups was shown. *, p<0.05; **, p<0.01. (b) C57/BL6 mice were inoculated with MC38 tumor cells (0.5×10e⁶ per mice) at day 0, and followed by the treatment with anti-FGL1, anti-LAG3, or control antibodies every four days from day 6 to day 18. In some groups, mice were also treated with one single dose of anti-B7-H1 (10B5) at day 6. Mean tumor diameter in indicated groups of mice at day 22 was shown.

### DETAILED DESCRIPTION

The general embodiments "comprising" or "comprised" encompass the more specific embodiment "consisting of". Furthermore, singular and plural forms are not used in a limiting way. As used herein, the singular forms "a", "an" and "the" designate both the singular and the plural, unless expressly stated to designate the singular only.

Any references made herein to methods of treatment refer to the compounds, pharmaceutical compositions and medicaments of the present invention for use in a method for treatment of the human body by therapy or for diagnosis.

The term "protein" as used herein is used interchangeably with "amino acid residue sequence" or "polypeptide" and refers to polymers of amino acids of any length. These terms also include proteins that are post-translationally modified through reactions that include, but are not limited to, glycosylation, acetylation, phosphorylation, glycation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with the same properties. The term "polypeptide" typically refers to a sequence with more than 10 amino acids and the term "peptide" means sequences with up to 10 amino acids in length. However, the terms may be used interchangeably.

The term "gene" as used herein refers to a DNA locus of heritable genomic sequence which affects an organism's trait by being expressed as a functional product or by regulation of gene expression. Genes and polynucleotides may include introns and exons as in genomic sequence, or just the coding sequences as in cDNAs, such as an open reading frame (ORF), comprising a start codon (methionine codon) and a translation stop codon. Genes and polynucleotides can also include regions that regulate their expression, such as transcription initiation, translation and transcription termination. Thus, also included are regulatory elements such as a promoter.

The terms "enhanced", "increased" and "elevated" are used interchangeably herein and refer to an increase by at least about 10% as compared to a control, for example an increase by at least about 20%, or at least about 30%, or at least about 40%, or at least about 50%, or at least about 75%, or at least about 80%, or at least about 90%, or at least about 100%, or at least about 200%, or at least about 300%, or any integer decrease between 10-300% as compared to a control. As used herein, a "control" is a reference value or a reference sample for base line expression from at least one subject not having cancer or a non-cancerous tissue sample, wherein the non-cancerous tissue sample preferably originates from the same organ as the cancer and more preferably from the cancer patient.

The methods and sample types used for establishing a cut-off value of a marker like FGL1 and for measuring the FGL1 level in a sample obtained from an individual or patient to be analysed match each other or are the same. Cut-off values, i.e., values above which elevated expression or overexpression (e.g., elevated expression of FGL1 compared to a control) is acknowledged can be obtained in a control group. The control group on which the cut-off value is based is chosen to match the group of individuals/patients under investigation.

A "vector" is a nucleic acid that can be used to introduce a heterologous polynucleotide into a cell. One type of vector is a "plasmid", which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA or RNA segments can be introduced into the viral genome.

The term "encodes" and "codes for" refers broadly to any process whereby the information in a polymeric macromolecule is used to direct the production of a second molecule that is different from the first. In other aspects, a DNA molecule can encode an RNA molecule (e.g., by the process of transcription that uses a DNA-dependent RNA polymerase enzyme). Also, an RNA molecule can encode a polypeptide, as in the process of translation. When used to describe the process of translation, the term "encode" also extends to the triplet codon that encodes an amino acid. In some aspects, an RNA molecule can encode a DNA molecule, e.g., by the process of reverse transcription incorporating an RNA-dependent DNA polymerase. In another aspect, a DNA molecule can encode a polypeptide, where it is understood that "encode" as used in that case incorporates both the processes of transcription and translation.

The term "allele" refers to any one of the different forms of a gene, genetic target region or generally DNA sequence at a single locus, i.e., chromosomal location. This includes coding sequences, noncoding sequences and regulatory sequences. Different alleles within a genome are not necessarily identical in nucleotide sequence.

The term "antibody" refers to a protein consisting of one or more polypeptides substantially encoded by immunoglobulin genes. The recognized immunoglobulin genes include the kappa, lambda, alpha, gamma, delta, epsilon and mu constant regions genes as well as the myriad immunoglobulin variable region genes. The terms "antibody" and "immunoglobulin" are used interchangeably herein. The term "antibody" is used herein in its broadest sense and encompasses monoclonal antibodies (including full length monoclonal antibodies), polyclonal antibodies, chimeric antibodies, humanized antibodies, human antibodies and multispecific antibodies (e.g. bispecific antibodies). The term "antibody" also encompasses antibody fragments, such as e.g. Fv, Fab, Fab', F(ab)2 or other antigen-binding subsequences of antibodies, i.e. antigen-binding fragments of original or "full length" antibodies. The term "antibody" also encompasses engineered molecules such as Nanobodies(R), i.e. humanized VHH domains derived from camelid antibodies, as well as "domain antibodies" or "single domain antibodies", i.e. immunoglobulins that can bind their antigen via one single immunoglobulin domain (and not by two paired VH/VL domains). The term "antibody" further encompasses antibody conjugates. Classical 4-chain, full-length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chains of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The term "antibody" further refers to a type of antibody comprising a plurality of individual antibodies having the same specificity (variable domain) and having the same constant domains.

The term "immune checkpoint inhibitory antibody" refers to an antibody as defined above binding to an inhibitory receptors (IRs) or more broadly interfering with the interaction between an inhibitory receptor and its ligand, wherein the inhibitory receptor is essential to balance co-stimulatory receptor activity and limit T-cell activation. Thus, such an antibody targets immune system checkpoints such as the cytotoxic T-lymphocyte antigen 4 (CTLA-4), the programmed death-1 (PD-1) or its ligand (PD-L1). Examples of immune checkpoint inhibitory antibodies suitable for use in the present invention, without being limited thereto, are anti-PD-1 antibodies, anti-PD-L1 antibodies, anti-IDO antibodies, anti-CTLA-4 antibodies, anti-Tim-3 antibodies, anti-GITR antibodies, anti-OX40 antibodies or anti-LAG3 antibodies. Moreover, an anti-FGL1 antibody may be regarded as an immune checkpoint inhibitor. In most cases the immune checkpoint inhibitory antibody is an antagonistic or blocking antibody, i.e., a blocking antibody selected from the group consisting of an anti-PD-1 antibody, an anti-PD-L1 antibody, an anti-IDO antibody, an anti-CTLA-4 antibody, an anti-Tim-3 antibody, an anti-LAG3 antibody, an anti-VISTA antibody, and an anti-BTLA antibody. However, an immune checkpoint inhibitory antibody may also be an agonistic antibody, such as an anti-GITR or an anti-OX40 antibody. With regard to the anti-LAG3 antibody, this antibody may be an antibody selectively inhibiting the interaction of LAG3 and MHC class II molecule or an antibody selectively inhibiting the interaction of LAG3 and FGL1 or an antibody inhibiting the interaction of LAG3 with FGL1 and MHC class II molecule. Preferably the immune checkpoint inhibitory antibody is an IgG antibody, more preferably a human or a humanized IgG antibody, even more preferably human or a humanized IgG1 or IgG4 antibody.

A "fusion protein" is defined as a protein which contains the complete sequences or any parts of the sequences of two or more originally separate natural or modified heterologous proteins or a composition of complete sequences or any parts of the sequences of two or more originally separate natural or modified heterologous proteins. Fusion proteins can be constructed by genetic engineering approaches by fusing the two or more genes, or parts thereof, that originally encode the two or more originally separate natural or heterologous proteins, or parts thereof. This results in a fusion protein with functional properties derived from each of the original proteins. Fusion proteins include, but are not limited to, Fc fusion proteins. A fusion protein as used herein may also be a Flag Fusion protein. Fusion proteins such as an FGL1 fusion protein or a LAG3 fusion protein may also be used in the methods of treatment or for use in treating cancer as described herein instead or in addition to the immune inhibitory antibodies. Preferably the fusion protein is an FGL1-Fc (FGL1-lg) or a LAG3-Fc (LAG3-Ig) protein.

The term "cytokine" refers to small proteins, which are released by cells and act as intercellular mediators, for example influencing the behavior of the cells surrounding the secreting cell. Cytokines may be secreted by immune, such as T-cells, B-cells, NK cells and macrophages, or other cells. Cytokines may be involved in intercellular signaling events, such as autocrine signaling, paracrine signaling and endocrine signaling. They may mediate a range of biological processes including, but not limited to immunity, inflammation, and hematopoiesis. Cytokines may be chemokines, interferons, interleukins, lymphokines or tumor necrosis factors.

The terms "expression" or "expressing" as used herein refer to transcription and/or translation of a nucleic acid sequence within a host cell. The level of expression of a gene product of interest in a cancer or a cancer patient may be determined on the basis of either the amount of corresponding mRNA that is present in a sample provided, or the amount of protein encoded by the selected gene, particularly by the gene encoding FGL1. For example, RNA transcribed from a gene, such as the FGL1 gene, can be quantified by Northern blot hybridization, ribonuclease RNA protection, in situ hybridization to cellular RNA or by PCR, such as qPCR. Proteins encoded by a gene, such as the FGL1 gene, can be quantitated by various methods, e.g. by ELISA, by Western blotting, by radioimmunoassay, by immunoprecipitation, immunohistochemistry, by testing for the biological activity of the protein, by immunostaining of the protein followed by FACS analysis or by homogeneous time-resolved fluorescence (HTRF) assays.

The term "the cancer patient has an elevated FGL1 expression" as used herein refers to a patient having an FGL1 expressing cancer, particularly a cancer with elevated FGL1 expression, more particularly a cancer with elevated FGL1 expression compared to a control. The FGL1 expression can be detected or determined in a sample of the cancer, such as in a biopsy of the cancerous tissue. However, the FGL1 expression may also be detected or determined in a blood sample of the cancer patient, such as a plasma or serum sample. FGL1 may be present in a bound and/or free (soluble) form. Thus, the term "the cancer patient has an elevated FGL1 expression" encompasses that the patient has a cancer having elevated FGL1 expression and/or that the patient has elevated FGL1 levels in blood. Thus, in one embodiment the patient has a cancer having elevated FGL1 expression, particularly compared to a control, such as a non-cancerous reference sample of the same tissue of said patient or in a reference sample of the same tissue of at least one reference subject, preferably reference samples of the same tissue of more than one reference subject or a reference value. The reference value may be based on a reference sample of the same tissue of at least one reference subject, preferably reference samples of the same tissue of more than one reference subject. The at least one reference subject is preferably a subject that does not have cancer. Enhanced expression may be detected as increased signal or increased percentage of cells positive for FGL1 (tumor proportion score (TPS)) in a tissue sample. In another embodiment or in addition to the previous embodiment the patient has elevated FGL1 expression, particularly elevated FGL1 levels, more particularly elevated FGL1 protein levels in blood, such as in serum and/or plasma. Preferably the FGL1 expression is elevated compared to control, such as in a non-cancerous reference blood sample, such as serum or plasma sample of at least one reference subject, preferably reference blood samples of more than one reference subject or a reference value. The reference value may be based on a reference blood sample of at least one reference subject, preferably reference blood samples of more than one reference subject. The at least one reference subject is preferably a subject that does not have cancer. Preferably, the reference subject does further not have liver injury. In one embodiment the patient has an FGL1 expressing cancer, wherein the cancer is not a liver cancer. Preferably the patient has a cancer with elevated FGL1 expression, wherein the cancer is not a liver cancer, more particularly a cancer with elevated FGL1 expression compared to a control, wherein the cancer is not a liver cancer.

The term "gene product" refers to both the RNA polynucleotide and polypeptide that is encoded by a gene or a DNA polynucleotide.

The term "sample" as used herein refers to a tissue sample or a bodily fluid sample, such as a blood sample. A tissue sample is a section of an organ or a tissue of the body, which typically includes several cell types, optionally with cytoskeletal structures that hold the cells together. A tissue sample can be obtained by a biopsy, for example, including by cutting, slicing, or a punch. It involves extraction of sample cells or tissue for examination. However, the terms "providing a sample", "a sample from a patient" or "a sample obtained from a patient" do not include the active step of extraction of the tissue or blood, but rather refer to the provision of an already extracted sample, i.e., the provision of an ex *vivo* sample from a patient. Further, a tissue sample may be a tumor sample or the respective control sample, preferably from the same tissue. Preferably the tissue sample is used as a formalin-fixed, paraffin-embedded (FFPE) sample. A blood sample may be a serum or plasma sample and is preferably a plasma sample. However, a blood sample may also include the cell fraction, e.g., for complete blood count and blood cell exam, such as a pheripheral blood smear. Other bodily fluid samples in addition to blood include without being limited thereto, mucous, seminal fluid, saliva, sputum, bronchial lavage, breast milk, bile and urine. A sample may further be a bone marrow biopsy or aspirate or a cerebrospinal fluid. The sample may be analysed for example, without being limited thereto, by cytochemistry, such as chemical stains (dyes) that react with certain substances found in or on different kind of cells, flow cytometry and immunohistochemistry (IHC), such as by using antibody staining, fluorescent in situ hybridization (FISH), Polymerase chain reaction (PCR) or ELISA. The term "a sample obtained from a patient" as uses herein refers to the sample *ex vivo,* i.e., after collection, and is synonymous to "providing a sample from a patient" or "providing an ex *vivo* sample from a patient".

The term "detecting FGL1 expression", "determining FGL1 expression" as used herein refers to measuring FGL1 expression levels by any means or methods known to the person skilled in the art, wherein FGL1 expression may be determined on a protein or mRNA level. Preferably the means or methods to measure FGL1 expression are able to quantitatively detect or determine FGL1 expression, preferably human FGL1 expression. Quantitatively detecting or determining FGL1 expression includes detecting or determining relative or absolute amounts. Methods suitable for quantitatively detecting and determining FGL1 expression include contacting the sample with an anti-FGL1 antibody and detecting the binding between FGL1 and the antibody, such as by ELISA or by IHC, or detecting the amount of mRNA encoding FGL1 protein in the sample, such as by PCR. In a specific embodiment detecting or determining FGL1 expression or elevated FGL1 expression involves administering an anti-FGL1 antibody to the patient and in vivo imaging, particularly, if the antibody is labelled, such as radioactively, fluorescently or otherwise.

The term "fibrinogen-like protein 1" abbreviated as FGL1 (synonyms LFIRE-1, HFREPI, Hepassocin, HP-041) refers to a protein of the fibrinogen family of proteins, which also includes fibrinogen-like protein 2 and clotting factors V, VIII, and XIII. However, the term "FGL1 expression" relates to both, protein and mRNA expression in humans unless otherwise stated. FGL1 is encoded by the *FGL1* gene. FGL-1 contains a fibrinogen related domain in its C-terminal portion, which contains the four conserved cysteines that are common to all members of the fibrinogen family. However, FGL-1 lacks the platelet-binding site, cross-linking region, and thrombin-sensitive site which allow the other members of the fibrinogen family to aid in fibrin clot formation. FGL1 is upregulated in regenerating liver and is abundantly associated with the fibrin matrix after clot formation. While the majority of FGL1 is found in plasma, approximately 20% of FGL1 remains in the serum after blood coagulation. Human FGL1 precursor has the amino acid sequence as provided by the NCBI Reference Sequence database under ID number NP_004458.3, wherein amino acids 1 to 22 correspond to the signal peptide and amino acids 23 to 312 correspond to the mature protein. The C-terminal globular domain of fibrinogen corresponds to amino acids 78 to 304.

The term "lymphocyte-activation gene 3 protein" abbreviated as LAG3, as used herein refers to an inhibitory molecule involved in the regulation of T cell activation, proliferation and homeostasis. It is also referred to as CD223. LAG3 is a CD4-related activation-induced cell surface molecule that acts as an immune checkpoint receptor. LAG3 is expressed on activated T cells, natural killer cells (NK cells), B cells and plasmacytoid dendritic cells (pDCs). The protein negatively regulates proliferation, activation and homeostasis in a similar fashion to CTLA-4 and PD-1. It has been reported to play a role in Treg suppressive function and helps maintaining CD8+ T cell in a tolerogenic state. LAG3 is structurally related to CD4 and binds with high affinity to MHC class II molecules. A single lysine residue (K468) within a conserved "KIEELE" motif is essential for interaction with downstream signaling molecules. It has four extracellular Ig-like domains, with conserved structural motifs between the D1 and D3 domains as well as the D2 and D4 domains. The first domain is an Ig-like V-type domain and the following three domains are Ig-like C2-type domains. The human LAG3 has approximately 70% homology with murine LAG3. A unique distinguishing feature of LAG3 is a proline-rich 30-aa loop between the C and C' β strands of the D1 domain. Although both, human and murine LAG-3, possess this loop, their homology is lowest in this region. Human LAG3 has the amino acid sequence as provided by the NCBI GenBank database under ID number AAH52589.

The term "immune checkpoint inhibitory antibody specifically inhibiting the interaction of FGL1 with LAG3" refers to an antibody as defined above binding to human FGL1 or human LAG3, thereby blocking the binding of FGL1 to LAG3. This blocking or antagonistic activity of the antibody may be without being limited thereto due to binding to the binding pocket (or binding interface) of FGL1 for LAG3 or alternatively of LAG3 for FGL1 or due to any other sterical interaction with FGL1 to LAG3 binding. With regard to the anti-LAG3 antibody, this antibody may be an antagonistic antibody selectively inhibiting the interaction of LAG3 and FGL1 or an antagonistic antibody inhibiting the interaction of LAG3 with FGL1 and MHC II. Preferably the immune checkpoint inhibitory antibody specifically inhibiting the interaction of FGL1 with LAG3 is an IgG antibody, more preferably a human or a humanized IgG antibody, even more preferably a human or a humanized IgG1 antibody.

The term "programmed death-1 (PD-1)" as used herein relates to an immune checkpoint that limits excessive immune responses to antigens and prevents autoimmunity. PD-1 can be expressed on a variety of immune cells including T cells, B cells, natural killer T (NKT) cells, activated monocytes and dendritic cells (DCs). It is expressed on the surface of activated T cells, but not resting T cells and has two known ligands, PD-L1 and PD-L2. PD-L1 is expressed on activated T cells, DCs, monocytes and myeloid cells and outside the hematopoietic system PD-L1 is expressed in lung, vascular endothelium, liver non-parenchymal cells, placental syncytiotrophoblast, and keratinocytes. A number or agents targeting PD-1 or PD-L1 interaction are in clinical development in various phases. Nivolumab and pembrolizumab are humanized PD-1 blocking antibodies that have already received approval from the U.S. Food and Drug Administration (FDA) and the European Medical Agency (EMA) for unresectable or advanced malignant melanoma (MM), non-small cell lung cancer (NSCLC) with progression on or after platinum-based chemotherapy and recurrent or metastatic squamous cell carcinoma of the head and neck (SCCHN) with progression on or after platinum-based chemotherapy. Nivolumab has further been approved for the treatment of advanced renal cell carcinoma (RCC) progressing after a previous therapy and relapsed or progressive classical Hodgkin lymphoma after autologous hematopoietic stem cell transplantation (HSCT). Pembrolizumab has further been approved for the first-line treatment of patients with 50 percent or more PD-L1 expression (TPS ≥ 50%). Atezolizumab is a fully humanized PD-L1 blocking antibody that has been approved by the FDA and the EMA for patients with locally advanced or metastatic urothelial carcinoma (UC) after prior platinum-containing chemotherapy or who are considered cisplatin ineligible and for patients with locally advanced or metastatic non-small cell lung cancer (NSCLC) after prior chemotherapy. Further anti-PD-L1 antibodies are durvalumab and avelumab.

### Uses of immune checkpoint inhibitory antibodies

In the present invention a method for treating cancer in a human patient is provided comprising administering to the patient an effective amount of an immune checkpoint inhibitory antibody specifically inhibiting the interaction of FGL1 with LAG3, and/or of an anti-FGL1 antibody (in the latter case, independent of whether a direct inhibition of interaction of FGL1 with LAG3 can be observed or not).

Also described is a method for treating cancer in a human patient comprising administering to the patient an effective amount of an immune checkpoint inhibitory antibody, preferably an antibody specifically inhibiting the interaction of FGL1 with LAG3, and/or of an anti-FGL1 antibody, wherein the cancer patient has an elevated FGL1 expression, preferably wherein the cancer patient has been determined to have elevated FGL1 expression. In a specific embodiment a sample obtained from the patient has been determined to have elevated FGL1 expression. The elevated FGL1 expression is typically elevated compared to a control. The method may comprise determining FGL1 expression in a sample obtained from the patient, and administering an effective amount of the immune checkpoint inhibitory antibody specifically inhibiting the interaction of FGL1 with LAG3, or of the anti-FGL1 antibody to the patient. The method may further comprise determining if FGL1 expression is elevated in a sample obtained from the patient, and if FGL1 expression is elevated, administering an effective amount of the immune checkpoint inhibitory antibody specifically inhibiting the interaction of FGL1 with LAG3 to the patient, or an effective amount of the anti-FGL1 antibody.

Also provided is an immune checkpoint inhibitory antibody specifically inhibiting the interaction of FGL1 with LAG3, or an anti-FGL1 antibody, for use in treating cancer in a human patient. In particular embodiments the cancer patient has an elevated FGL1 expression, preferably the cancer patient has been determined to have elevated FGL1 expression. In specific embodiments a sample from the patient has been determined to have elevated FGL1 expression. The elevated FGL1 expression is typically elevated compared to a control. Preferably, the elevated FGL1 expression has been determined in a sample from the patient *in vitro.* The antibody for use may comprise determining FGL1 expression in a sample from the patient, before the immune checkpoint inhibitory antibody specifically inhibiting the interaction of FGL1 with LAG3, or the anti-FGL1 antibody, is to be administered to the patient. The antibody for use may further comprise determining if FGL1 expression is elevated in a sample from the patient, and if FGL1 expression is elevated, administering the immune checkpoint inhibitory antibody specifically inhibiting the interaction of FGL1 with LAG3, or the anti-FGL1 antibody, to the patient.

In another aspect a method for treating cancer in a human patient is described comprising administering to the patient an effective amount of an antibody binding to human FGL1. Also provided is an antibody binding to human FGL1 for use in treating cancer in a human patient. In particular embodiments the cancer patient has an elevated FGL1 expression, preferably the cancer patient has been determined to have elevated FGL1 expression. In specific embodiments a sample from the patient has been determined to have elevated FGL1 expression. The elevated FGL1 expression is typically elevated compared to a control. Preferably the FGL1 antibody inhibits interaction of FGL1 with LAG3.

The method or the antibody for use may further comprise a) identifying a patient in need of treatment of cancer, b) determining that the patient has elevated FGL1 expression, and c) administering an effective amount of the immune checkpoint inhibitory antibody specifically inhibiting the interaction of FGL1 with LAG3, or of the anti-FGL1 antibody to the patient. The methods or the antibody for use may further comprise determining that FGL1 expression is elevated in a sample from the patient. The methods or the antibody for use may further comprise providing a sample from the patient to determine the FGL1 expression.

The invention further relates to the method of treating cancer in a human patient or the antibody for use, comprising a) optionally providing a sample from the patient, b) measuring FGL1 expression level in said sample, c) comparing FGL1 expression to a control, d) identifying the patient as likely responsive to treatment with the immune checkpoint inhibitory antibody specifically inhibiting the interaction of FGL1 with LAG3, or with the anti-FGL1 antibody, when FGL1 expression level has been measured as elevated compared to the control, and e) administering an effective amount of the immune checkpoint inhibitory antibody specifically inhibiting the interaction of FGL1 with LAG3, or of the anti-FGL1 antibody, to the patient.

The present invention further relates to a method of selecting a therapy comprising an immune checkpoint inhibitory antibody specifically inhibiting the interaction of FGL1 with LAG3, or an anti-FGL1 antibody, for a cancer patient, the method comprising measuring FGL1 expression level in a sample obtained from the patient and selecting said therapy for the patient when the FGL1 expression levels is elevated.

The FGL1 expression according to the methods or the antibody for use of the invention is preferably elevated compared to control. The control may be a reference value or a reference sample for base line expression from at least one subject not having cancer or a non-cancerous tissue sample, wherein the non-cancerous tissue sample preferably originates from the same organ as the cancer and more preferably from the cancer patient. The sample from the patient as used in the methods or the uses according to the invention is preferably a tissue sample and/or a blood sample, preferably a tumor or a blood sample. Thus, in one embodiment the patient has a cancer having elevated FGL1 expression, particularly compared to a control, such as a non-cancerous reference sample of the same tissue of said patient or in a reference sample of the same tissue of at least one reference subject, preferably reference samples of the same tissue of more than one reference subject or a reference value. The reference value may be based on a reference sample of the same tissue of at least one reference subject, preferably reference samples of the same tissue of more than one reference subject. The at least one reference subject is preferably a subject that does not have cancer. Enhanced expression may be detected as increased signal or increased percentage of cells positive for FGL1 (tumor proportion score (TPS)). In another embodiment or in addition to the previous embodiment the patient has elevated FGL1 expression, particularly elevated FGL1 levels, more particularly elevated FGL1 protein levels in blood, such as in serum or plasma. Preferably the FGL1 expression is elevated compared to control, such as in a reference blood sample, such as a serum or a plasma sample of at least one reference subject, preferably reference blood samples of more than one reference subject or a reference value. The reference value may be based on a reference blood sample of at least one reference subject, preferably reference blood samples of more than one reference subject. The at least one reference subject is preferably a subject that does not have cancer. Preferably, the reference subject does further not have liver injury. In one embodiment the patient has an FGL1 expressing cancer, wherein the cancer is not a liver cancer. Preferably the patient has a cancer with elevated FGL1 expression, wherein the cancer is not a liver cancer, more particularly a cancer with elevated FGL1 expression compared to a control, wherein the cancer is not a liver cancer. A tissue or tumor sample may be used as a formalin-fixed paraffin-embedded sample for FGL1 detection.

The immune checkpoint inhibitory antibody specifically inhibiting the interaction of FGL1 with LAG3 may be an anti-LAG3 antibody, or an anti-FGL1 antibody, or combinations thereof. In a specific embodiment the immune checkpoint inhibitory antibody is an anti-LAG3 antibody, wherein the anti-LAG3 antibody inhibits the interaction of FGL1 with LAG3. In another specific embodiment the immune checkpoint inhibitory antibody is an anti-FGL1 antibody. In yet another specific embodiment, the immune checkpoint inhibitory antibody is an anti-LAG3 antibody and an anti-FGL1 antibody. Preferably, the anti-LAG3 and the anti-FGL1 antibody inhibit the interaction of FGL1 with LAG3. In particular embodiments the anti-FGL1 antibody is used for treating cancer in a human patient.

The immune checkpoint inhibitory antibody specifically inhibiting the interaction of FGL1 with LAG3, and/or the anti-FGL1 antibody used in the method or the treatment according to the invention may also be used in addition with at least one further immune checkpoint inhibitory antibody (the term again including antigen-binding fragments thereof), wherein the further immune checkpoint inhibitory antibody may be any checkpoint inhibitory antibody other than the immune checkpoint inhibitory antibody specifically inhibiting the interaction of FGL1 with LAG3, or the anti-FGL1 antibody, respectively. Preferably the at least one further immune checkpoint inhibitory antibody is selected from the group consisting of an anti-PD-1 antibody, an anti-PD-L1 antibody, an anti-IDO antibody, an anti-CTLA-4 antibody, an anti-Tim-3 antibody, an anti-BTLA antibody, an anti-VISTA antibody, an anti-GITR antibody, an anti-OX40 antibody and an anti-LAG3 antibody. Particularly, wherein the anti-LAG3 antibody selectively inhibits the interaction of LAG3 with MHC class II molecule. More preferably the at least one immune checkpoint inhibitory antibody is an anti-CTLA-4, an anti-PD-1 antibody, or an anti-PD-L1 antibody and even more preferably the at least one immune checkpoint inhibitory antibody is an anti-PD-1 antibody or an anti-PD-L1 antibody.

The immune checkpoint inhibitory antibody and the at least one further immune checkpoint inhibitory antibody can be administered to the patient systemically (e.g., orally, parenterally, subcutaneously, intravenously, rectally, intramuscularly, intraperitoneally, intranasally, transdermally, or by inhalation or intracavitary installation), topically, or by application to mucous membranes, such as the nose, throat and bronchial tubes. Preferably, the immune checkpoint inhibitory antibody and the at least one further immune checkpoint inhibitory antibody is administered intravenously or subcutaneously. In case more than one immune checkpoint inhibitory antibody is used, each immune checkpoint inhibitory antibody may be administered independently by a suitable route of administration, preferably intravenously or subcutaneously. The immune checkpoint inhibitory antibody and the at least one further immune checkpoint inhibitory antibody may be administered simultaneously or separate from each other. Typically, they are provided in separate formulation or dosage forms, which may be combined prior to administration or administered separately.

In certain embodiments, the cancer treated with an immune checkpoint inhibitory antibody specifically inhibiting the interaction of FGL1 with LAG3, or with an anti-FGL1 antibody, includes but is not limited to, a solid tumor, a hematological cancer (e.g., leukemia, lymphoma, myeloma, e.g., multiple myeloma), and a metastatic lesion. In one embodiment, the cancer is a solid tumor. Examples of solid tumors include malignancies, e.g., sarcomas and carcinomas, e.g., adenocarcinomas of the various organ systems, such as those affecting the lung, breast, ovarian, lymphoid, gastrointestinal (e.g., colon), anal, genitals and genitourinary tract (e.g., renal, urothelial, bladder cells, prostate), pharynx, CNS (e.g., brain, neural or glial cells), head and neck, skin (e.g., melanoma), and pancreas, as well as adenocarcinomas which include malignancies such as colon cancers, rectal cancer, renal-cell carcinoma, liver cancer, non-small cell lung cancer, cancer of the small intestine and cancer of the esophagus. Preferably the cancer is a brain cancer, a colorectal cancer, a melanoma, a prostate cancer or a lung cancer. More preferably the cancer is a lung cancer or a prostate cancer, even more preferably, the lung cancer is a non-small cell lung cancer (NSCLC). The treatment may be further accompanied by chemotherapy or radiotherapy. The cancer may be at an early, intermediate, late stage or metastatic cancer. In a specific embodiment the cancer is not a liver cancer, such as a hepatocarcinoma, with or without a viral infection, e.g., a chronic viral hepatitis.

In one embodiment, the cancer is chosen from a lung cancer (e.g., a non-small cell lung cancer (NSCLC) (e.g., a NSCLC with squamous and/or non-squamous histology, or a NSCLC adenocarcinoma), a melanoma (e.g., an advanced melanoma), a renal cancer (e.g., a renal cell carcinoma), a myeloma (e.g., a multiple myeloma), a prostate cancer, a breast cancer (e.g., a breast cancer that does not express one, two or all of estrogen receptor, progesterone receptor, or Her2/neu, e.g., a triple negative breast cancer), a colorectal cancer, a pancreatic cancer, a head and neck cancer (e.g., head and neck squamous cell carcinoma (HNSCC)), anal cancer, gastro-esophageal cancer, thyroid cancer, cervical cancer, a lymphoproliferative disease (e.g., a post-transplant lymphoproliferative disease), a brain cancer or a hematological cancer, T-cell lymphoma, B-cell lymphoma, a non-Hodgkin lymphoma, or a leukemia (e.g., a myeloid leukemia or a lymphoid leukemia).

In another embodiment, the cancer is chosen from a carcinoma (e.g., advanced or metastatic carcinoma), melanoma or a lung carcinoma, e.g., a non-small cell lung carcinoma. In one embodiment, the cancer is a lung cancer, e.g., a non-small cell lung cancer or small cell lung cancer, preferably a non-small cell lung cancer. In one embodiment, the cancer is a melanoma, e.g., an advanced melanoma. In one embodiment, the cancer is an advanced or unresectable melanoma that does not respond to other therapies. In another embodiment, the cancer is a prostate cancer, e.g., an advanced prostate cancer. In yet another embodiment, the cancer is a myeloma, e.g., multiple myeloma. In yet another embodiment, the cancer is a renal cancer, e.g., a renal cell carcinoma (RCC) (e.g., a metastatic RCC or clear cell renal cell carcinoma (CCRCC)).

In certain embodiments the patient to be treated for cancer may have a primary or acquired resistance to at least one checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-BTLA antibody, an anti-VISTA antibody, an anti-GITR antibody, and an anti-OX40 antibody. In some embodiments the patient has a primary or acquired resistance to PD-1/PD-L1 checkpoint inhibition. A patient having a primary (innate) resistance to a checkpoint inhibitor may also be referred to as non-responder. However, in some cases late relapses are observed, which indicates acquired (secondary) resistance to the treatment

Preferably, the cancer patient has, or is identified as having elevated FGL1 expression. In certain embodiments, the cancer patient has, or is identified as having, a tumor that has elevated FGL1 expression. In some embodiments, the methods described herein further include identifying a cancer patient suitable for treatment with an immune checkpoint inhibitory antibody based on having elevated FGL1 expression or a tumor having elevated FGL1 expression. In some embodiments, the cancer patient has, or is identified as having, a high percentage of cancer cells that are positive for FGL1.

In some embodiments, the methods described herein further include identifying a cancer patient based on having an elevated FGL1 expression or having a high percentage of cancer cells that are FGL1 positive and one or more of a lung cancer, e.g., squamous cell lung cancer or lung adenocarcinoma; a head and neck cancer; a squamous cell cervical cancer; a stomach cancer; an esophageal cancer; a thyroid cancer; a melanoma, a brain cancer, a colorectal cancer, a prostate cancer and/or a nasopharyngeal cancer (NPC), preferably a brain cancer, a colorectal cancer, a melanoma, a prostate cancer or a lung cancer. Methods and antibodies disclosed herein are useful for treating metastatic lesions having elevated FGL1 expression and being associated with the aforementioned cancers.

Also provided is a method for treating cancer in a human patient comprising administering to the patient an effective amount of an immune checkpoint inhibitory antibody specifically inhibiting the interaction of FGL1 with LAG3, or of an anti-FGL1 antibody, wherein the cancer patient has an elevated FGL1 expression determined according to any one of the diagnostic methods described herein.

If the antibody of the invention is an antibody that binds to FGL1, preferably to human FGL1, the antibody may or may not directly inhibit the binding of FGL1 to (human) LAG3. The antibody may or may not compete with human LAG3 for binding to FGL1. Although the molecular mechanisms via which such non-competing antibody is exercising its immunomodulatory effect are not fully clear, such antibody may nevertheless have the same therapeutic effect as anti-FGL1 antibodies competing with LAG3 for binding to FGL1.

In yet another aspect an antibody that binds to human LAG3 (anti-LAG3), wherein the antibody inhibits interaction of human LAG3 with human FGL1 is provided.

Preferably the anti-FGL1 antibodies and/or the anti-LAG3 antibodies described herein are human or humanized antibodies, more preferably human or humanized IgG1 or IgG4 antibodies.

### Diagnostic methods

Provided herein is a method of detecting elevated FGL1 expression in a human cancer patient, said method comprises providing a sample from the patient; and detecting FGL1 expression in the sample, wherein the quantitatively detected FGL1 expression in the sample is preferably compared to a control, wherein the method may optionally further comprise identifying the patient as likely susceptible to the treatment with an immune checkpoint inhibitory antibody specifically inhibiting the interaction of FGL1 with LAG3, or to the treatment with an anti-FGL1 antibody, when the FGL1 expression has been determined as being elevated compared to the control.

Also provided herein is a method of assessing susceptibility to the treatment with an immune checkpoint inhibitory antibody in a cancer patient, comprising a) providing a sample from the patient, b) detecting FGL1 expression in the sample, c) comparing the FGL1 expression determined in step (b) to a control, wherein the FGL1 expression in the sample is elevated compared to a control, and optionally further comprising identifying the patient as likely susceptible to the treatment with an immune checkpoint inhibitory antibody specifically inhibiting the interaction of FGL1 with LAG3, or with an anti-FGL1 antibody, when the FGL1 expression has been determined as being elevated compared to the control.

Also provided is a method of categorizing a tumor of a patient, comprising (a) providing a sample from the patient, (b) detecting FGL1 expression in the sample, and (c) identifying the tumor as a candidate for the treatment with an immune checkpoint inhibitory antibody specifically inhibiting the interaction of FGL1 with LAG3, or with an anti-FGL1 antibody, wherein the method optionally further comprises as step of comparing the FGL1 expression determined in step (b) to a control, wherein an elevated FGL1 expression in the sample compared to a control identifies the tumor as a candidate for such treatment. At the same time, an elevated FGL1 expression level in the sample may also indicate that the tumor has evaded treatment with another immune checkpoint inhibitory antibody, such as an anti-PD-1 antibody, an anti-PD-L1 antibody, an anti-IDO antibody, an anti-CTLA-4 antibody, an anti-Tim-3 antibody, an anti-BTLA antibody, an anti-VISTA antibody, an anti-GITR antibody and an anti-OX40 antibody. In such case, combined use of such other immune checkpoint inhibitory antibody together with an immune checkpoint inhibitory antibody specifically inhibiting interaction of FGL1 with LAG3, or together with an anti-FGL1 antibody, may overcome the primary or acquired resistance to such other immune checkpoint inhibitory antibody and thus may overcome the poor prognosis associated with elevated FGL1 expression.

Also provided is a method of predicting the responsiveness of a human cancer patient comprising (a) providing a sample from the patient, (b) measuring FGL1 expression level in said sample, (c) comparing FGL1 expression to a control, and (d) identifying the patient as likely responsive to treatment with an immune checkpoint inhibitory antibody specifically inhibiting the interaction of FGL1 with LAG3, or with an anti-FGL1 antibody, when FGL1 expression level has been determined to be elevated compared to the control, or identifying the patient as less likely responsive when FGL1 expression has not been determined to be elevated compared to the control.

Also provided is a method of predicting the responsiveness of a human cancer patient comprising (a) providing a sample from the patient treated or previously treated with at least one immune checkpoint inhibitory antibody, (b) determining FGL1 expression level in said sample, (c) comparing FGL1 expression to a control, and (d) identifying the patient as less likely responsive to treatment with said at least one immune checkpoint inhibitory antibody when FGL1 expression level has been determined as elevated compared to the control. Wherein the at least one immune checkpoint inhibitory antibody is an immune checkpoint inhibitory antibody other than an immune checkpoint inhibitory antibody specifically inhibiting the interaction of FGL1 with LAG3 and other than an anti-FGL1 antibody, but preferably selected from the group consisting of an anti-PD-1 antibody, an anti-PD-L1 antibody, an anti-IDO antibody, an anti-CTLA-4 antibody, an anti-Tim-3 antibody, an anti-GITR antibody and an anti-OX40 antibody. More preferably the antibody is selected from the group consisting of an anti-CTLA-4, an anti-PD-1 antibody, and an anti-PD-L1 antibody, more preferably the antibody is an anti-PD-1 antibody or an anti-PD-L1 antibody. Poor prognosis associated with elevated FGL1 expression may be overcome by an add-on therapy with the immune checkpoint-inhibitory antibody specifically inhibiting interaction of FGL1 with LAG 3 or an anti-FGL1 antibody according to the invention.

In view of the various interactions between immune checkpoint pathways, enhanced or elevated FGL1 expression may also indicate that treatment of such patient with any immune checkpoint inhibitor, including an (antagonistic) anti-PD-1 antibody, anti-PD-L1 antibody, anti-LAG3 antibody, anti-IDO antibody, anti-CTLA-4 antibody, anti-Tim-3 antibody, anti-GITR antibody, anti-OX40 antibody, or anti-VISTA antibody, may bring about therapeutic effects in the treatment of a cancer disease. Thus, there are provided methods of treatment, methods of predicting the responsiveness of a human cancer patient, methods of assessing susceptibility of a tumor or cancer disease, methods of categorizing a tumor of a patient, and the like, wherein enhanced or elevated FGL1 expression is used as an indicator that such patient, tumor or cancer disease can be treated by an immune checkpoint inhibitor, such as e.g. an (antagonistic) anti-PD-1 antibody, anti-PD-L1 antibody, anti-LAG3 antibody, anti-IDO antibody, anti-CTLA-4 antibody, anti-Tim-3 antibody, anti-VISTA antibody, anti-GITR antibody, anti-OX40 antibody, or anti-VISTA antibody as mentioned above.

The method of detecting elevated FGL1 expression, the method of assessing susceptibility, the method of categorizing and the method of predicting the responsiveness described herein are preferably *in vitro* methods. Further the step of detecting FGL1 expression particularly involves quantitatively detecting FGL1 expression or FGL1 expression levels, preferably in a sample from the patient. The methods may further comprise or be followed by a step of administering to the cancer patient the immune checkpoint inhibitory antibody specifically inhibiting the interaction of FGL1 with LAG3 or of the anti-FGL1 antibody. The sample is preferably a tissue and/or a bodily fluid sample, preferably a tissue and/or a blood sample, more preferably a tumor and/or a blood sample. The FGL1 expression may be quantitatively detected in the sample for instance (a) by contacting the sample with an anti-FGL1 antibody and detecting the binding between FGL1 (FGL1 protein) and the antibody, or (b) detecting the amount of mRNA encoding FGL1 in the sample. Additionally, FGL1 copy number gains may be determined. In one embodiment the control is a reference value or a reference sample for base line expression from at least one reference subject not having cancer or a non-cancerous tissue sample, wherein the non-cancerous tissue sample preferably originates from the same organ as the cancer and more preferably from the cancer patient.

The FGL1 expression as detected according to the methods is preferably elevated compared to control. The control may be a reference value or a reference sample for base line expression from at least one reference subject not having cancer or a non-cancerous tissue sample, wherein the non-cancerous tissue sample preferably originates from the same organ as the cancer and more preferably from the cancer patient. The sample from the patient as used in the methods described herein is preferably a tissue sample and/or a blood sample, preferably a tumor and/or a blood sample.

Thus, in one embodiment the patient has a cancer having elevated FGL1 expression, particularly compared to a control, such as a non-cancerous reference sample of the same tissue of said patient or in a reference sample of the same tissue of at least one reference subject, preferably reference samples of the same tissue of more than one reference subject or a reference value. The reference value may also be referred to as cut-off value. The reference value may be based on a reference sample of the same tissue of at least one reference subject, preferably reference samples of the same tissue of more than one reference subject. The at least one reference subject is preferably a subject that does not have cancer. Enhanced expression may be detected as increased signal or increased percentage of cells positive for FGL1 (tumor proportion score (TPS)).

In another embodiment or in addition to the previous embodiment the patient has elevated FGL1 expression, particularly elevated FGL1 levels, more particularly elevated FGL1 protein levels in blood, such as in serum or plasma. Preferably the FGL1 expression is elevated compared to control, such as in a reference blood sample, such as a serum or a plasma sample of at least one reference subject, preferably reference blood samples of more than one reference subject or a reference value. The reference value may be based on a reference blood sample of at least one reference subject, preferably reference blood samples of more than one reference subject. The at least one reference subject is preferably a subject that does not have cancer. Preferably, the reference subject does further not have liver injury.

In one embodiment the patient has an FGL1 expressing cancer, wherein the cancer is not a liver cancer. Preferably the patient has a cancer with elevated FGL1 expression, wherein the cancer is not a liver cancer, more particularly a cancer with elevated FGL1 expression compared to a control, wherein the cancer is not a liver cancer.

In specific embodiments the FGL1 expression is detected in a sample from a patient having a primary or acquired resistance to at least one checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, and an anti-OX40 antibody or from a patient having a primary or acquired resistance to PD-1/PD-L1 checkpoint inhibition.

A cancer patient having an elevated FGL1 expression may be a patient having an FGL1 expressing cancer, particularly a cancer with elevated FGL1 expression, more particularly a cancer with elevated FGL1 expression compared to a control. The FGL1 expression can be detected or determined in a sample of the cancer, such as in a biopsy of the cancerous tissue. However, the FGL1 expression may also be detected or determined in a blood sample of the cancer patient, such as a plasma or serum sample. FGL1 may be present in a bound and/or free (soluble) form.

In one embodiment the patient has a cancer having elevated FGL1 expression, particularly compared to a control, such as a non-cancerous reference sample of the same tissue of said patient or in a reference sample of the same tissue of at least one reference subject, preferably reference samples of the same tissue of more than one reference subject or a reference value. The reference value may be based on a reference sample of the same tissue of at least one reference subject, preferably reference samples of the same tissue of more than one reference subject. The at least one reference subject is preferably a subject that does not have cancer. Enhanced expression may be detected in a tumor sample as increased signal (staining intensity) and/or increased percentage of cells positive for FGL1 (tumor proportion score (TPS)). Typically the sample is in the form of a formalin-fixed, paraffin-embedded tissue samples, e.g., as tissue microarray (TMA) sections. The percentage of tumor cells with FGL1 staining to assess the patient's TPS is between 0% and 100%. The TPS is the percentage of viable tumor cells showing partial or complete staining relative to all viable tumor cells present in the sample (positive and negative). Infiltrating immune cells, normal cells, necrotic cells and debris must be excluded from scoring. A minimum of 100 viable tumor cells is needed to determine FGL1 expression. The FGL1 expression level of the specimen can be interpreted as "no FGL1 expression: TPS<1%", "FGL1 expression: TPS ≥1%", "high FGL1 expression:TPS ≥50%". Other relevant factors may be expression levels (staining intensity). Staining may be performed using in situ hybridization, preferably fluorescent in situ hybridization (FISH), or preferably immunohistochemistry (IHC) using methods known in the art. For IHC, sections of formalin-fixed, paraffin-embedded tissue samples typically need to be deparaffinized and rehydrated. After antigen retrieval endogenous peroxidases should be quenched, e.g., with hydrogen peroxide before reacting with an anti-FGL1 antibody or a LAG3 fusion protein. A further relevant factor may also be gene copy number gains (additional gene copy in the genome), as may be identified using FISH (Inoue, Y. et al., Clinical significance of PD-L1 and PD-L2 copy number gains in non-small-cell lung cancer, Oncotarget, 7(22): 32113-32128). Further, circulating-free tumor DNA from plasma, such as derived from EDTA anti-coagulated peripheral whole blood may be quantitatively detected. In addition to primary tumor samples, FGL1 expression may also be determined in metastatic regional lymph node samples. Lymph nodes may also be analyzed in case of a patient with lymphoma or leukemia.

Nucleic acid hybridization simply involves contacting a probe (e.g., an oligonucleotide or larger polynucleotide) and target nucleic acid under conditions where the probe and its complementary target can form stable hybrid duplexes through complementary base pairing. As used herein, hybridization conditions refer to standard hybridization conditions under which nucleic acid molecules are used to identify similar nucleic acid molecules. Such standard conditions are disclosed, for example, in Sambrook et al. (1989) Molecular Cloning: A Laboratory Manual. Cold Spring Harbor Labs Press (see specifically, pages 9.31-9.62). In addition, formulae to calculate the appropriate hybridization and wash conditions to achieve hybridization permitting varying degrees of mismatch of nucleotides are disclosed, for example, in Meinkoth et al. (1984) Anal. Biochem. 138, 267-284. Nucleic acids that do not form hybrid duplexes are washed away from the hybridized nucleic acids and the hybridized nucleic acids can then be detected, typically through detection of an attached detectable label. Nucleic acids are denatured by increasing the temperature or decreasing the salt concentration of the buffer containing the nucleic acids. Under low stringency conditions (e.g., low temperature or high salt or both) hybrid duplexes (e.g., DNA:DNA, RNA:RNA, or RNA:DNA) will form even where the annealed sequences are not perfectly complementary. Thus specificity of hybridization is reduced at lower stringency. Conversely, at higher stringency (e.g., higher temperature or lower salt) successful hybridization requires fewer mismatches.

In another embodiment or in addition to the tissue sample the patient has elevated FGL1 expression, particularly elevated FGL1 levels, more particularly elevated FGL1 protein levels in blood, such as in serum and/or plasma. Preferably the FGL1 expression is elevated compared to control, such as in a non-cancerous reference blood sample, such as serum or plasma sample of at least one reference subject, preferably reference blood samples of more than one reference subject or a reference value.

The reference value may be based on a reference blood sample of at least one reference subject, preferably reference blood samples of more than one reference subject. The at least one reference subject is preferably a subject that does not have cancer. Preferably, the reference subject does further not have liver injury. FGL1 expression in serum and/or plasma may be detected, e.g., using enzyme-linked immunosorbent assay (ELISA) with an anti-FGL1 antibody or a LAG3 fusion protein as detecting agent and are known in the art. Concentrations of FGL1 in serum are in the ng/ml range. The hybridized nucleic acids or antibodies are detected by detecting one or more labels attached to the sample nucleic acids or antibodies. The labels may be incorporated by any of a number of means well known to those of skill in the art. Suitable detectable labels include any composition detectable by spectroscopic, photochemical, biochemical, immunochemical, electrical, optical or chemical means. Useful labels include fluorescent dyes (e.g., fluorescein, Texas red, rhodamine, Alexa fluors, Spectrum dyes, and the like), quantum dots, radiolabels (e.g., ³H, ¹²⁵I, ³⁵S, ¹⁴C, or ³²P), and colorimetric labels. Means of detecting such labels are well known to those of skill in the art. Thus, for example, radiolabels may be detected using photographic film or scintillation counters, fluorescent markers may be detected using a photodetector to detect emitted light and fluorescence microscopes or flow cytometry. Colorimetric labels are detected by simply visualizing the colored label. Preferably, the hybridizing nucleic acids are detected by fluorescent labels and most preferably, in the context of a fluorescence in situ hybridization (FISH) assay. FISH assays are well known in the art. Antibodies are preferably detected by fluorescent labels or colorimetric labels.

In one embodiment the patient has an FGL1 expressing cancer, wherein the cancer is not a liver cancer. Preferably the patient has a cancer with elevated FGL1 expression, wherein the cancer is not a liver cancer, more particularly a cancer with elevated FGL1 expression compared to a control, wherein the cancer is not a liver cancer. The cancer patient suitable for the methods of the invention has a cancer as specified for the therapeutic uses of the immune checkpoint inhibitory antibodies.

### Methods for screening

Also provided herein is a method for screening for an immune checkpoint inhibitory antibody comprising
(a) providing a FGL1 protein, preferably comprising at least the fibrinogen domain of human FGL1;
(b) providing a LAG3 protein, preferably comprising at least domains 1 and 2 of human LAG3;
(c) contacting the FGL1 protein and the LAG3 protein in the presence of an antibody to be screened;
(d) determining binding of the FGL1 protein to the LAG3 protein; and
(e) identifying the antibody to be screened as an immune checkpoint inhibiting antibody that specifically inhibits interaction of the FGL1 protein with the LAG3 protein, if the binding of the FGL1 protein and the LAG3 protein is reduced compared to the binding of the FGL1 protein and the LAG3 protein in the absence of said antibody to be screened. Preferably, the antibody is a human or humanized antibody.

Further provided herein is a method for screening for a small molecule that inhibits the binding of (human) FGL1 to (human) LAG3, comprising
(a) providing a FGL1 protein, preferably comprising at least the fibrinogen domain of human FGL1;
(b) providing a LAG3 protein, preferably comprising at least domains 1 and 2 of human LAG3;
(c) contacting the FGL1 protein and the LAG3 protein in the presence of a small molecule, e.g. from a compound library, e.g. in the format of a high throughput screen,
(d) determining binding of the FGL1 protein to the LAG3 protein in the presence and in the absence of said small molecule; and
(e) identifying a small molecule that specifically inhibits binding of said FGL1 protein to said LAG3 protein as a FGL1-LAG3 checkpoint inhibitor molecule, suitable for developing a drug for the treatment of cancer diseases as mentioned hereinbefore.

Furthermore, described herein are small molecules identified by a method as set out above to be inhibitors for the binding of (human) FGL1 to (human) LAG3, and which thus can be used as drugs, or can be developed into drugs, that are acting on such immune checkpoint pathway, and thereby can be used in the (immuno)therapy of cancer diseases as mentioned hereinbefore.

In particular embodiments binding of soluble proteins of FGL1 (e.g., full length human FGL1 or labelled FGL1 such as 3xFLAG tagged FGL1 protein) to LAG3 (e.g., a fusion protein of human LAG3, such as LAG3-Fc fusion protein) are measured. The binding or interaction may be measured e.g., using a biosensor, such as an Octet instrument.

In other embodiments at least one of the proteins comprises a transmembrane domain to facilitate membrane expression and the other binding partner is a soluble protein. Human LAG3 is a transmembrane protein and therefore contains a transmembrane domain. FGL1 is a soluble protein that may be fused with an artificial transmembrane domain (ATM) such as from FIBCD1 gene (ATM insertion into the N terminal) or B7-H6 gene (ATM insertion into the C terminal) to facilitate membrane expression. Preferably the FGL1 fused with an artificial transmembrane domain is transfected into cells over-expressing several immune-associated adaptors, such as DAP10, DAP12, FcRgamma, and CD3epsilon, that facilitate membrane expression, e.g., into 293T.2A cells. The soluble protein may be FGL1, optionally labelled with a detectable label, such as a tag (e.g., a 3xFLAG tag), an Fc domain or a fluorescent label, or may be detected using a secondary antibody. Alternatively, the soluble protein may be a soluble fusion protein of LAG3, such as an LAG3-Fc fusion protein, and may either be directly labelled or detected with a secondary antibody or any other secondary reagent known in the art. In a preferred embodiment the FGL1 protein comprises amino acids 78 to 304 or amino acids 23 to 312 of the amino acid sequence as provided by NCBI Reference Sequence database under ID number NP_004458.3. Methods for determining protein-protein interactions are known in the art and may include, without being limiting thereto, the use of a cellular detection system (CDS), fluorescent microscopy and flow cytometry.

### Kits

In yet another aspect, a kit for selecting a cancer patient that would benefit from an immune checkpoint inhibitory antibody is described, wherein the kit comprises means for quantitatively detecting FGL1 expression in a sample from a cancer patient, and a control. The control may be selected from the group consisting of (a) a reference sample for detecting enhanced FGL1 expression, (b) a reference sample for detecting base line FGL1 expression, (c) instructions containing a predetermined reference level of FGL1 expression that has been correlated with susceptibility to an immune checkpoint inhibitory antibody, (d) instructions containing a predetermined reference level of FGL1 expression that has been correlated with not being susceptible to an immune checkpoint inhibitory antibody, and/or combinations of (a), (b), (c) or (d). In one embodiment the means for quantitatively detecting FGL1 expression in a sample from a cancer patient are (a) an antibody specific for FGL1, (b) a primer pair specific for FGL1 and optionally a probe hybridizing to the amplified product, and/or a probe hybridizing to the FGL1 target sequence, preferably the FGL1 mRNA target sequence.

### EXAMPLES

### Methods

### Animals

C57BL/6 (B6) mice at 6-8 weeks old were purchased from Charles River (Wilmington, MA). FGL1 conditional knockout mice were obtained from European Mouse Mutant Archive (EMMA), and homologous targeting of this mouse strain was performed in a genetically engineered C57/BL6 ES cell (Agouti JM8A). FGL1 whole body knockout mice (*Fgl1-*/*-,* herein referred to as FGL1-KO) were generated by crossing with CMV-Cre mice (Jackson Lab). All mouse protocols were in accordance with NIH guidelines and were approved by the Animal Care and Use Committee of Yale University School of Medicine.

### Plasmids, fusion proteins, and antibodies

The human cDNA library coding ~6500 full-length plasma membrane genes and ~1300 soluble genes were collected from Genecopeia (Rockville, MD), Open Biosystems (Huntsville, AL), or were individually cloned. All of the genes used for the ultra-high throughput receptor array described below were cloned into a mammalian expression vector. Human and mouse fusion proteins were generated by tagging the relevant genes coding the extracellular domain with human Fc or 3xFLAG, expressed in 293T cells and purified by affinity column. The domain deletion plasmids for FGL1 and LAG3 were produced by PCR or GeneArt synthesis (Thermo Fisher, Waltham, MA). Antibodies used in flow cytometry and *in vitro* studies were purchased from BD Biosciences (San Jose, CA), BioLegend (San Diego, CA), or Thermo Fisher (Waltham, MA). The hybridoma for anti-LAG3 monoclonal antibody (C9B7W; directed against mouse LAG3) was from Dr. Dario Vignali (University of Pittsburgh). Rat mAbs against mouse FGL1, and mouse mAbs against human FGL1 were generated by animal immunization with FGL1 fusion protein.

### Ultra-high throughput receptor array technology

Other than the plasma membrane genes, soluble genes were selected based on their expression profiles, homology with known immune-related molecules (Bioinformatics analysis using BioGPS, Immgen, HPM, UCSC genome browser and NCBI databases), and later fused with artificial transmembrane domain (ATM) from FIBCD1 gene (ATM insertion into the N terminal) or B7-H6 gene (ATM insertion into the C terminal) to facilitate membrane expression. To construct each receptor array plate, cDNA library plasmids were diluted individually in OptiMEM (4 ug/ml) before transfer into 384 deep-well plates (VWR), with 200 ul plasmid solution in each well. Two microliters (ul) of plasmid solution from each well of the four 384 plates was transferred into 1536-well plates (Greiner) by a robotic liquid handling system (PlateMate, Thermo Fisher). The entire human receptor array contained five 1536 plates. Unless specified, these 1536 plates were further foiled and stored at -80°C. For the receptor array screening, the array plates were brought out from -80°C and placed in an incubator until the solution was completed thawed. The array plates were spun down (600 g, 2 min) and dispensed with 2 ul OptiMEM containing lipofecamine 3000 (7 ul lipofectamine per ml) per well with Multidrop Combi robotic dispenser (Thermo Fisher) and quickly shook for 1 min by an ultra-speed orbital shaker. All plates were stored at room temperature for 20 min and followed by the addition of 2x10e3 293T.2A cells (293T cells over-expressing several immune-associated adaptors, DAP10, DAP12, FcRy, and CD3ε, that facilitate membrane expression) in 4 ul DMEM medium per well, by Multidrop Combi. The plates were further spun down (1000 g, 4 min) to dispose of bubbles inside each well before incubation at 37°C. Eighteen hours after transfection, 2 ng of LAG3-Fc fusion protein (human or mouse) and 3 ng of anti-Fc secondary antibody were added into each well. These plates were read 6-8 hrs later using the Applied Biosystems 8200 cellular detection system (CDS) and analyzed using the CDS 8200 software. Human Fc Receptors served as internal positive controls within each plate for screened fusion proteins.

### Protein-protein interaction analysis

Protein interactions were measured and analyzed on an Octet Red instrument (PALL, NY), performed at room temperature. The Octet Protein A sensor was dipped into solution containing mouse or human LAG3-Fc fusion protein (10 ug/ml) and subsequently loaded with different concentrations of 3xFLAG tagged FGL1 proteins (2 fold serial dilutions starting from 10 ug/ml). Protein association and disassociation was monitored and analyzed by Octet software.

### In vitro T cell function assay

Anti-mouse CD3 mAb (eBiosciences) was pre-coated in 96-well flat plates at the indicated concentrations. Mouse splenocytes from C57/BL6 mice or LAG3-KO mice were added into each well at 3x10e5/well in the presence of FGL1-Fc fusion protein or isotype-matched control Fc at 5 ug/ml and cultured for 3 days. ³H-TdR was added before the final 16 hrs of culture and thymidine incorporation was counted with a MicroBeta Trilux liquid scintillation counter (PerkinElmer, Waltham, MA).

For antigen-specific T cell experiments, 3A9 T cell hybridoma, or 3A9 over-expressing mouse LAG3 full-length gene was incubated with LK35.2 cell line in the presence of HEL peptide (1.5 ug/ml). The indicated concentration of FGL1 fusion protein or control was also included for testing the *in vitro* functions. In some groups, anti-FGL1 or anti-LAG3 antibodies (5 ug/ml) were applied to monitor the antagonistic effect. The IL-2 levels in the supernatant 24 hrs after the cell co-culture were analyzed by CBA kit (BD Pharmingen).

### In vivo tumor experiments

8 to 10-week-old LAG3-KO, FGL1-KO mice, or control littermates were used for *in vivo* tumor experiments. Tumors were inoculated by single subcutaneous injection of MC38 or Hepa1-6 cells on day 0 (5×10⁵ cells for MC38 and 1×10⁶ for Hepa1-6), and mice were treated intraperitoneally with 100 ug of anti-FGL1 or anti-LAG3 (C9B7W) antibodies on days 6, 10, 14 and 18, with saline or rat Ig as a control. For anti-PD-L1 (B7-H1) combinational therapy, mice were further treated with a hamster mAb against PD-L1 (clone 10B5; 100 ug) at day 6. Tumor growth was monitored by electronic caliper twice a week and measured using mean tumor diameter: (length + width)/2.

### Bioinformatics

The microarray data on solid cancer lesions and the counterpart normal tissues (254 datasets in total covering 14 types of human cancers) were collected from Oncomine (Thermo Fisher), FGL1 upregulation or downregulation frequency in the datasets of each cancer (fold change> 3 for upregulation, or <0.3 for downregulation, and P value <0.05 as cutoff) were further analyzed by the program R. Moreover, FGL1 expression in individuals from several cancers compared to the counterpart normal tissues were performed by utilizing TCGA cancer databases. The original microarray data was normalized by cancer browser (https://genome-cancer.ucsc.edu/) and then analyzed by R.

### Histology and Immunohistochemistry

Tissues were removed from naive mice or tumor bearing mice, embedded in paraffin, cut into 5 µm thick sections for H&E or immunohistochemistry staining. For FGL1 staining, tissues were deparaffinized and rehydrated prior to antigen retrieval. Tissue sections were then stained with anti-FGL1 antibody (177R4), followed by incubation with an amplification system (DakoCytomation, Glostrup, Denmark).

### Statistical Analysis

Student's t-test and two-way anova was used for statistical analysis, and P values reflect comparison within the control samples. P values less than 0.05 were considered statistically significant. The error bars in figures represent Standard Error of the Mean (SEM).

### Example 1

Lymphocyte-activation gene 3 protein (LAG3) is a cell surface T-cell suppressive protein and is believed to function via interacting with major histocompatibility complex class II (MHC-II). This interaction, however, is not required for LAG3-induced suppressive function in various experimental settings, suggesting the presence of an additional functional LAG3 ligand. Using a genome-scale receptor array system, we identified fibrinogen like protein 1 (FGL1), a liver-enriched soluble protein with hepatocyte mitogenic activity and metabolic functions, as a LAG3 binding partner.

A genome-scale Receptor Array Platform (RAP) was employed to search for new LAG3 binding protein using Immunoglobulin (Ig) Fc-tagged LAG3 extracellular domain fusion proteins (Fig.1a). The RAP is a semi-automatic large-scale gene expression system in which individual human cDNA encoding transmembrane and soluble proteins can be transiently over-expressed on the surface of 293T cells (Yao, S. et al. B7-h2 is a costimulatory ligand for CD28 in human. Immunity 34, 729-740, (2011)). The current version of the RAP includes over 90% of all annotated genes coding human transmembrane (~6,500) and soluble (~1,300). To facilitate the membrane expression, several immune adaptor genes (DAP10, DAP12, FcRγ, and CD3ε) were engineered into the 293T cells, and genes encoding soluble proteins were fused to an artificial transmembrane domain allow their expression on cell surface (Fig. 1a). Also, the original platform in a 384-well plate format was also modified to a 1,536-well plate format for better efficiency. FGL1, a secreted protein, was shown to be a major binding protein for LAG3-Fc in the RAP screening.

### Example 2

The interaction appeared to be conserved across species in human and mouse (Figure 1d and e). The FGL1/LAG3 interaction could be validated by flow cytometry (Fig. 2b) and in the Octet bio-layer interferometry with a ~1.5 nM Kd (Fig. 1d), indicating a high affinity interaction. A slow disassociation rate in both mouse and human binding indicates possible stability of this interaction (Figure 1c and f). Other FGL1 homologs, including FGL2, which is implicated in the modulation of Treg functions, as well as several angiopoietin related family members, did not show interaction with LAG3 (data not shown), indicating the highly specific nature of the FGL1/LAG3 interaction.

We sought to determine the binding domain of FGL1 to LAG3 and whether this would affect MHC-II interaction. FGL1 is composed of a coil-coil domain (CCD) and a fibrinogen-like domain (FD) (Yamamoto, T. et al. Molecular cloning and initial characterization of a novel fibrinogen-related gene, HFREP-1. Biochem Biophys Res Commun 193, 681-687, (1993)). Through domain deletion experiments, we demonstrated that the FD, not CCD, is responsible for LAG3 binding (Fig. 2a and c). LAG3 protein consists of four Ig-like domains (D1-D4), a transmembrane domain (TM), and an intracellular domain (IC) (Huard, B. et al. Characterization of the major histocompatibility complex class II binding site on LAG-3 protein. Proc Natl Acad Sci U S A 94, 5744-5749, (1997); Triebel, F. et al. LAG-3, a novel lymphocyte activation gene closely related to CD4. J Exp Med 171, 1393-1405, (1990)) (Fig. 2b). The deletion of D3-D4 domain in LAG3 did not affect FGL1 binding, while either D1 or D2 alone partially decreased the binding (Fig. 2b and d), suggesting that both D1 and D2 contribute to the FGL1/LAG3 interaction. Previous studies demonstrated that a single point mutation (Y73F) in the C' strand of D1 domain of mouse LAG3 (corresponding to Y77F in human LAG3) disrupted MHC class II binding (Huard, B. et al. Characterization of the major histocompatibility complex class II binding site on LAG-3 protein. Proc Natl Acad Sci U S A 94, 5744-5749, (1997); Workman, C. J., Dugger, K. J. & Vignali, D. A. Cutting edge: molecular analysis of the negative regulatory function of lymphocyte activation gene-3. J Immunol 169, 5392-5395, (2002)). Interestingly, we found that FGL1-Fc bound to the LAG3 Y73F mutant as strong as LAG3 WT (Fig. 2b and d). Furthermore, we utilized an anti-LAG3 mAb (C9B7W clone), reported to bind D2 domain of mouse LAG3 without disrupting LAG3/MHC class II interaction (Andrews, L. P., Marciscano, A. E., Drake, C. G. & Vignali, D. A. LAG3 (CD223) as a cancer immunotherapy target. Immunol Rev 276, 80-96, (2017); Workman, C. J., Rice, D. S., Dugger, K. J., Kurschner, C. & Vignali, D. A. Phenotypic analysis of the murine CD4-related glycoprotein, CD223 (LAG-3). Eur J Immunol 32, 2255-2263, (2002); Cemerski, S., Zhao, S., Chenard, M., Laskey, J., Cui, L., Shukla, R., Haines, B., Hsieh, E., Beaumont, M., Mattson, J., Blumenschein, W., Hirsch, H., Fayadat-Dilman, L., Liang, L., De Waal Malefyt, R., T cell activation and anti-tumor efficacy of anti-LAG-3 antibodies is independent of LAG-3 - MHCII blocking capacity. Journal for Immunotherapy of Cancer 3(Suppl 2), 183, (2015)). After pre-incubation of 293T LAG3⁺ cells with C9B7W, we found a complete abrogation of FGL1/LAG3 binding (Fig. 1g). These data suggest that FGL1 interacts with LAG3 in a large area involving both D1 and D2.

To directly test whether FGL1 and MHC class II competed for LAG3 binding, we cultured LAG3+ cells with MHC class II I-Ab fusion protein in combination with high concentrations of FGL1 (up to 100-fold increase), no significant competition was observed (data not shown). These data suggest that FGL1 interacts via the fibrinogen C-terminal region with LAG3 in a large area involving both D1 and D2 that is non-redundant to MHC class II binding.

### Example 3

In a competition assay it was further determined whether existing monoclonal anti-LAG3 antibodies block FGL1-LAG3 interactions. CHO cells expressing human LAG3 or primary T cells activated with an anti-CD3 antibody to induce LAG3 expression were stained with fluorescently labeled FGL1 (1 ug/ml FGL-A647) in the presence or absence of anti-LAG3 antibodies (10 ug/ml anti-LAG3-A488 antibodies; R&D Systems) to determine whether they compete for FGL1 binding to LAG3. Binding of FGL1 was not reduced in the presence of anti-LAG3 antibodies (data not shown) and therefore do not seem to interfere with FGL1 :LAG3 interaction.

### Example 4

Next, we analysed the function of the FGL1/LAG3 interaction on T cells *in vitro.* LAG3 is highly expressed on T cells after activation. FGL1-Fc fusion protein bound activated T cells from WT but not LAG3 KO mice as analysed by flow cytometry (data not shown). FGL1-Fc suppressed splenic T-cell proliferation upon anti-CD3 stimulation, but the suppressive activity was lost in LAG3-KO splenocytes (Fig. 3a). Similarly, FGL1 fusion protein was able to suppress the antigen specific activation of murine 3A9-LAG3+ T cell line in a dose dependent fashion (Fig. 3b). Thus, FGL1 inhibits T cell proliferation and function *in vitro.* To further demonstrate the dependence of the FGL1/LAG3 pathway on T cell inhibition, we generated a specific anti-FGL1 mAb (clone 177R4) that blocks FGL1 fusion protein binding to LAG3 expressing 293T cells in a similar manner to anti-LAG3 (Figure 3c). Both antibodies abrogate the suppressing effect of FGL1 on IL-2 production from 3A9-LAG3 cells (Fig. 2d) and promote antigen-specific T cell activation *in vivo,* as determined by increased plasma levels of IFNgamma and TNF-alpha (Fig. 4a and b). Our results support that FGL1 and LAG3 function as a receptor/ligand pair that negatively regulates T cell responses.

Similar results were obtained in activated human primary T cells. CD4 T cells and CD8 T cells showed enhanced LAG3 expression when stimulated with anti-CD3 antibody at or above 1 ug/ml within 24 hours. LAG3 expression was maintained for at least 72 hours following anti-CD3 stimulation with 1 or 3 ug/ml as analyzed by flow cytometry (data not shown). LAG3 expression on activated primary T cells was further detectable by FGL 1-Fc staining (10 µg/ml) using T cells isolated from human PBMCs on day 4 following activation with anti-CD3/anti-CD28 coated beads (data not shown).

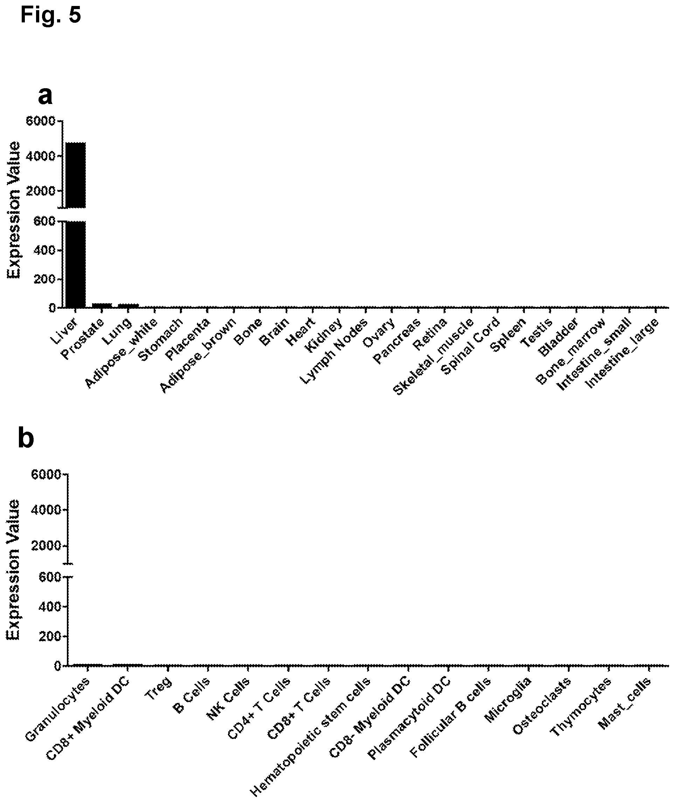

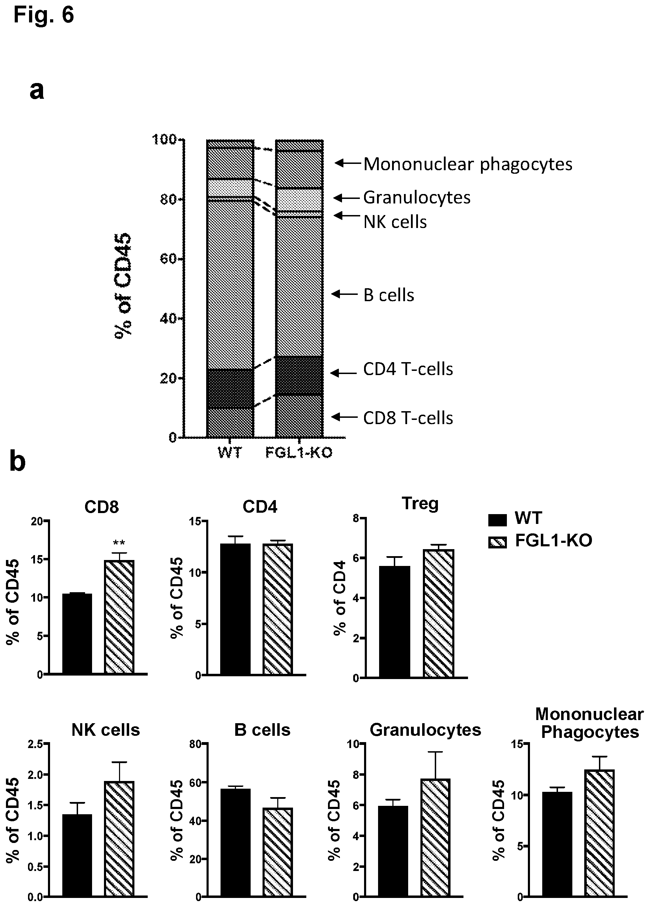

To evaluate the potential roles of FGL-1 in the regulation of the immune system, a FGL1 knockout (FGL1-KO) in the C57BL/6 background using an agouti color gene modified mouse embryonic stem cell line (JM8) was generated (Pettitt, S. J. et al. Agouti C57BL/6N embryonic stem cells for mouse genetic resources. Nat Methods 6, 493-495, (2009)). In wildtype (WT) mice, FGL1 is selectively expressed in the liver and not by the immune system (Fig. 5a) (Yan, J. et al. Cloning and characterization of a mouse liver-specific gene mfrep-1, up-regulated in liver regeneration. Cell Res 12, 353-361, (2002); Li, C. Y. et al. Recombinant human hepassocin stimulates proliferation of hepatocytes in vivo and improves survival in rats with fulminant hepatic failure. Gut 59, 817-826, (2010); Liu, Z. & Ukomadu, C. Fibrinogen-like protein 1, a hepatocyte derived protein is an acute phase reactant. Biochem Biophys Res Commun 365, 729-734, (2008)). The absence of liver FGL-1 expression by western blot and plasma FGL-1 levels by ELISA in the FGL1-KO mice compared to WT littermates was confirmed (data not shown). FGL1-KO mice have overall normal appearance, organ size, and litters, indicating that FGL1 does not globally affect the development and growth of mice. However, FGL1-KO have high incidence of dermatitis with obvious lymphocyte. 8 out of 20 FGL1 KO mice (12 ~ 16 months old) developed dermatitis, while only 1 out of 15 WT littermates developed dermatitis. Five out of eight 12-16-month-old females, but not males, had elevated anti-doublestranded DNA autoantibodies in sera (data not shown). Peripheral blood mononuclear cells analysis by mass cytometry (Bendall, S. C. et al. Single-cell mass cytometry of differential immune and drug responses across a human hematopoietic continuum. Science 332, 687-696, (2011)), showed a tendency to higher CD8 T cells frequency in FGL1 KO mice in comparison to FGL-1 WT, but not for CD4 T cells or other immune subsets (Fig. 6a and b). In contrast, there were no significant differences in T-cell subsets in peripheral tissues such as spleen or liver (data not shown). These data suggest that intrinsic FGL1 does not affect development and growth, while it may participate in regulating physiological immune responses.

### Example 5

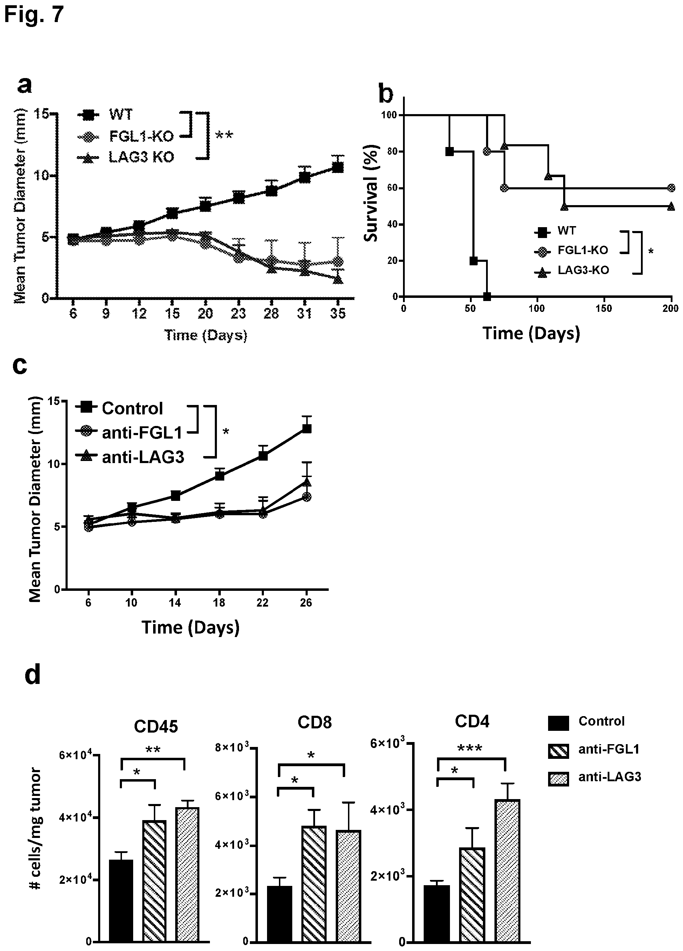

Given the promising role of LAG3 as a target in cancer immunotherapy and the newly identified role of the FGL1/LAG3 pathway in suppressing T cell immunity, we tested FGL1 deficiency or blockade in tumor immunity. Using the immunogenic MC38 mouse colon cancer model, we found that mice deficient in either FGL1 or LAG3 were resistant to transplanted tumors in the majority of the KO mice on day 35 in comparison with outgrowth of tumors in all wild type (WT) mice (Fig. 7a). While all of the WT mice reached endpoint (mean tumor diameter of 15 mm) within 60 days, -50% of the FGL1-KO or LAG3-KO mice were free of tumors beyond 150 days (Fig. 7b). Anti-FGL1 and anti-LAG3 (clone C9B7W) mAb also significantly inhibited the growth of subcutaneously implanted MC38 murine colon cancer (Fig. 7c) and Hepa1-6 murine liver cancer (data not shown) cell lines in syngeneic C57BL/6 mice. Depletion of either CD8+ or CD4+ T cells by specific mAbs eliminated the anti-tumor effect of both FGL1 and LAG3 mAb in the MC38 model (data not shown), suggesting that the anti-tumor effect was T-cell dependent. Through the tumor infiltrated leukocytes (TIL) analysis by mass cytometry, we observed a significant increase of absolute numbers of leukocytes (CD45+ cells), CD8 and CD4 T cells per mg of tumor in treated (anti-FGL1 or anti-LAG3) mice in contrast to control group (Fig. 7d).

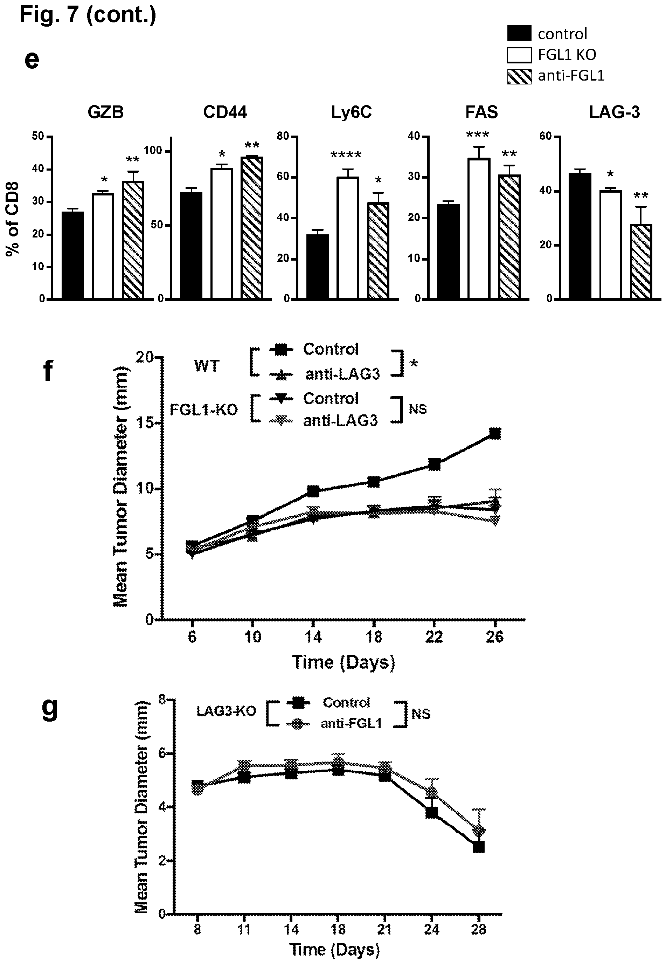

Also, a marked increase in effector memory T-cell populations in the tumor of treated vs. control mice was observed, but not in the tumor-draining lymph nodes and spleen (data not shown). When characterizing the functional changes of FGL1 deficiency or FGL1 blockade in TILs, a significant increase in functional markers (Granzyme B, CD44, Ly6C, FAS) and decrease in LAG-3 in both, FGL-1 KO and anti-FGL1 treated mice in comparison with the control group was observed (Fig. 7e).

To rigorously exclude the effect of other LAG3 ligand(s), we tested the anti-tumor effect of C9B7W in FGL1 KO mice. While anti-LAG3 mAb (C9B7W) clearly slowed down MC38 tumor growth in WT mice, this effect was eliminated in FGL1 KO mice (Fig. 7f). Therefore, the anti-tumor effect of anti-LAG3 mAb C9B7W is mediated via the blockade of FGL1, and not MHC class II or other LAG3 ligands. In addition, we found the effect of anti-FGL1 was also dependent on LAG3, as this antibody could not confer tumor resistance in LAG3-KO mice (Fig. 7g). Thus, our findings support a critical role of FGL1/LAG3 as a functional and interdependent pair in the control of anti-tumor immunity.

### Example 6

Given the relevance of FGL1/LAG3 in mouse cancer models, we explored the potential of FGL1 as a novel anti-tumor target in a clinical setting. In healthy individuals, FGL1 expression is largely limited to liver based on the tissue microarray database (Fig. 8a) and a recent reported human proteome analysis (Kim, M. S. et al. A draft map of the human proteome. Nature 509, 575-581, (2014)). The meta-analysis of the Oncomine databases revealed the upregulation of FGL1 mRNA in several solid tumors, with the highest expression (8/23, or 35%) in lung cancer datasets (Fig. 8b). Furthermore, FGL1 was demonstrated to be one of the most up-regulated genes in TCGA lung adenocarcinoma TCGA database and in many other cancers except liver cancer (Fig. 8c). To reliably measure FGL1, we validated an immunofluorescence assay using 293T cells transfected with FGL1, as well as tumoral and normal lung tissues, that were used to establish the threshold expression cut-point. FGL1 expression was then analyzed in 275 non-small cell lung cancer (NSCLC) cases by multiplex quantitative immunofluorescence (QIF), with paired normal lung tissue for 30 out of 275 cases. FGL1 expression was limited to the tumor cell compartment with minimal expression in the stroma and negative staining in normal paired lung tissue (data not shown). Among all the lung cancer cases, we found positive staining in ~15% of specimens (Fig. 9a), and tumors with high FGL1 expression in tumor biopsies were associated with worse prognosis (Fig. 9b). In addition, we also found higher plasma FGL1 levels in NSCLC patients compared to healthy donors in two independent cohorts (Cohort#1, from University of Navarra (Fig. 9c) and Cohort#2, from Fujian Medical University (data not shown)) as analyzed by ELISA. However, there was no difference of plasma FGL1 levels in patients with or without liver injury (ALT ≥ 40 U/L; ALT < 40 U/L) and in patients with or without metastasis (data not shown).

### Example 7

It was further tested whether the FGL1/LAG-3 pathway could be an alternative suppression pathway in the tumor microenvironment acting independently or in coordination with the B7-H1 (PD-L1)/PD-1 pathway. Therefore, we analyzed association of baseline plasma FGL1 levels with the efficacy of anti-PD-1 therapy in metastatic NSCLC patients (Cohort#1). Higher expression of FGL1 was associated with worse overall survival in patients treated with anti-PD-1 mAb (Fig. 9d). Similar results were observed in an independent cohort (Cohort#3 from Yale) of metastatic melanoma patients treated with anti-PD-1 mAb (Fig. 9e).

We further tested the role of anti-FGL1/anti-LAG3 in the context of anti-B7-H1 blockade by using the MC38 tumor model. Single-agent anti-B7-H1 mAb slowed down the tumor growth and improved survival, with death occurring within 90 days (Fig. 10a). Similarly, single-agent anti-FGL1 or anti-LAG3 mAb treatments failed to prolong survival beyond 3 months. Surprisingly, anti-B7-H1 mAb in combination with anti-FGL1 or anti-LAG3 mAb significantly improve survival time (Fig. 10a) and tumor burden (Fig. 10b) as compared with single-agent therapy. In addition, over 30% of mice were free of tumor for over 150 days (Fig. 10a). All these data together support a role of FGL1/LAG-3 pathway as a primary resistance mechanism in cancer patients treated with anti-PD1/PDL1 therapy. Consequently, either anti-FGL1 or anti-LAG3 blocking antibody potentiates T cell-mediated tumor immunity and suppresses tumor growth in several mouse models in a receptor-ligand interdependent manner, and synergizes with the B7-H1 (PD-L1)/PD-1 blockade therapy.

### Summary

Known anti-LAG3 therapy targets the interaction with its only known ligand, MHC class II. The inventors propose an alternative, independent LAG3 pathway involving FGL1 with potentially important implications for cancer immunotherapy. FGL1 inhibits T cell responses, and (antagonistic) anti-FGL1 or anti-LAG3 blocking antibody impairs tumor growth. Though FGL1 has limited expression in normal tissues, it is highly expressed and has important prognostic value in several human solid cancers, including lung cancer and melanoma. Given the high expression of FGL1 in primary resistance patients to anti-PD1/PD-L1 therapy, the FGL1/LAG3 pathway could be a potential mechanism for immune escape. Further the data suggest that FGL1 expression has prognostic value as a biomarker for successful treatment with drugs interfering with the FGL1/LAG3 immune checkpoint pathway, and as a biomarker for predicting anti-PD-1 responses, predicting poor prognosis in anti-PD1/PD-L1 therapy and potentially also other checkpoint inhibitory molecules.

## Claims

1. An antibody specifically inhibiting the interaction of fibrinogen-like protein 1 (FGL1) with lymphocyte-activation gene 3 protein (LAG3) for use in the treatment of cancer in a human patient.

2. The antibody for use according to claim 1, wherein the cancer patient has an elevated FGL1 expression compared to a reference value or a reference sample for base line expression from at least one subject not having cancer or a non-cancerous tissue sample, wherein the non-cancerous tissue sample preferably originates from the same organ as the cancer and more preferably from the cancer patient.

3. The antibody for use according to any one of claims 1 to 2, wherein said antibody specifically inhibiting the interaction of FGL1 with LAG3 is an anti-FGL1 antibody or an anti-LAG3 antibody.

4. An antibody specifically binding to human FGL1 for use in the treatment of cancer in a human patient.

5. The antibody for use according to any one of claims 1 to 4, wherein the antibody is to be used in combination with at least one further immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody and an anti-OX40 antibody.

6. The antibody for use according to any one of claims 1 to 5, wherein the patient has (i) a primary or acquired resistance to at least one checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody and an anti-OX40 antibody, or (ii) wherein the patient has a primary or acquired resistance to PD-1/PD-L1 checkpoint inhibition.

7. The antibody for use according to any one of claims 1 to 6, wherein the cancer is a brain cancer, a colorectal cancer, a melanoma, a prostate cancer or a lung cancer, preferably a non-small cell lung cancer (NSCLC).

8. The antibody for use according to any one of claims 1 to 7, wherein the antibody is to be used in combination with chemotherapy or radiotherapy.

9. A method of selecting a therapy for a cancer patient comprising an antibody specifically inhibiting the interaction of FGL1 with LAG3, the method comprising determining FGL1 expression level in a sample from the patient and selecting said therapy for the patient when the FGL1 expression levels is elevated compared to a reference value or a reference sample for base line expression from at least one subject not having cancer or a non-cancerous tissue sample, wherein the non-cancerous tissue sample preferably originates from the same organ as the cancer and more preferably from the cancer patient.

10. An in vitro method of categorizing a tumor of a patient and/or assessing susceptibility of a cancer patient to the treatment with an immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, an anti-OX40 antibody, an anti-FGL1 antibody, an anti-LAG3 antibody and preferably an antibody that specifically inhibits the interaction of FGL1 with LAG3, comprising
(a) providing a sample from the patient,
(b) detecting FGL1 expression in the sample, and
(c) comparing said FGL1 expression determined in step (b) to a control, wherein said FGL1 expression in said sample is elevated compared to said control, wherein the control is a reference value or a reference sample for base line expression from at least one subject not having cancer or a non-cancerous tissue sample, wherein the non-cancerous tissue sample preferably originates from the same organ as the cancer and more preferably from the cancer patient.

11. A method of predicting the responsiveness of a human cancer patient, comprising
(a) providing a sample from the patient,
(b) determining FGL1 expression level in said sample,
(c) comparing FGL1 expression to a control,
(d) identifying the patient as likely responsive to treatment with an immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, an anti-OX40 antibody, an anti-FGL1 antibody, an anti-LAG3 antibody and preferably an antibody that specifically inhibits the interaction of FGL1 with LAG3, when FGL1 expression level has been determined as elevated compared to the control, or identifying the patient as less likely responsive when FGL1 expression has been determined as not elevated compared to the control, wherein the control is a reference value or a reference sample for base line expression from at least one subject not having cancer or a non-cancerous tissue sample, wherein the non-cancerous tissue sample preferably originates from the same organ as the cancer and more preferably from the cancer patient.

12. An immune checkpoint inhibitory antibody selected from the group consisting of an anti-PD-1 antibody, an anti-PD-L1 antibody, an anti-IDO antibody, an anti-CTLA-4 antibody, an anti-Tim-3 antibody, an anti-GITR antibody, an anti-VISTA antibody, an anti-BTLA antibody, an anti-OX40 antibody, an anti-FGL1 antibody, an anti-LAG3 antibody and an antibody that specifically inhibits the interaction of FGL1 with LAG3, for use in the treatment of cancer in a human patient, wherein the cancer patient has an elevated FGL1 expression compared to a reference value or a reference sample for base line expression from at least one subject not having cancer or a non-cancerous tissue sample, wherein the non-cancerous tissue sample preferably originates from the same organ as the cancer and more preferably from the cancer patient.

13. A method for screening for an immune checkpoint inhibitory antibody or for a small molecule that inhibits the binding of FGL1 to LAG3, comprising
(a) providing a FGL1 protein, preferably comprising at least the fibrinogen domain of human FGL1;
(b) providing a LAG3 protein, preferably comprising at least domains 1 and 2 of human LAG3;
(c) contacting said FGL1 protein and said LAG3 protein in the presence of an antibody to be screened or of a small molecule, e.g. from a compound library, e.g. in the format of a high throughput screen;
(d) determining binding of said FGL1 protein to said LAG3 protein in the presence and in the absence of said immune checkpoint inhibitory antibody or of said small molecule; and
(e) identifying the antibody to be screened as an antibody that specifically inhibits interaction of said FGL1 protein with said LAG3 protein if the binding of said FGL1 protein and said LAG3 protein is reduced compared to the binding of said FGL1 protein and said LAG3 protein in the absence of said antibody to be screened or identifying a small molecule that specifically inhibits binding of said FGL1 protein to said LAG3 protein as a FGL1-LAG3 checkpoint inhibitor molecule.

14. An antibody that specifically binds to human FGL1, wherein the antibody inhibits binding of human FGL1 to human LAG3.

15. An antibody that specifically binds to human LAG3, wherein the antibody inhibits binding of human LAG3 to human FGL1, and wherein the antibody further inhibits interaction of human LAG3 with human MHC class II molecule.

16. An anti-FGL1 antibody for use in the treatment of cancer in a human patient, wherein the cancer patient has an elevated FGL1 expression compared to a reference value or a reference sample for base line expression from at least one subject not having cancer or a non-cancerous tissue sample, wherein the non-cancerous tissue sample preferably originates from the same organ as the cancer and more preferably from the cancer patient.

## Patentansprüche

1. Antikörper, der spezifisch die Wechselwirkung von Fibrinogen-ähnlichem Protein 1 (FGL1) mit Lymphocyte-activation gene 3-Protein (LAG3) inhibiert, zur Verwendung bei der Behandlung von Krebs bei einem menschlichen Patienten.

2. Antikörper zur Verwendung nach Anspruch 1, wobei der Krebspatient eine erhöhte FGL1-Expression im Vergleich zu einem Referenzwert oder einer Referenzprobe für die Baseline-Expression von mindestens einem Subjekt, das keinen Krebs hat, oder einer nicht krebsartigen Gewebeprobe aufweist, wobei die nicht krebsartige Gewebeprobe bevorzugt von dem gleichen Organ wie der Krebs und bevorzugter von dem Krebspatienten stammt.

3. Antikörper zur Verwendung nach einem der Ansprüche 1 bis 2, wobei der Antikörper, der spezifisch die Wechselwirkung von FGL1 mit LAG3 inhibiert, ein anti-FGL1-Antikörper oder ein anti-LAG3-Antikörper ist.

4. Antikörper, der spezifisch an humanem FGL1 bindet, zur Verwendung bei der Behandlung von Krebs bei einem menschlichen Patienten.

5. Antikörper zur Verwendung nach einem der Ansprüche 1 bis 4, wobei der Antikörper in Kombination mit mindestens einem weiteren Immuncheckpoint-inhibitorischen Antikörper, ausgewählt aus der Gruppe bestehend aus einem anti-PD-1-Antikörper, einem anti-PD-L1-Antikörper, einem anti-IDO-Antikörper, einem anti-CTLA-4-Antikörper, einem anti-Tim-3-Antikörper, einem anti-GITR-Antikörper, einem anti-VISTA-Antikörper, einem anti-BTLA-Antikörper und einem anti-OX40-Antikörper, zu verwenden ist.

6. Antikörper zur Verwendung nach einem der Ansprüche 1 bis 5, wobei der Patient (i) eine primäre oder erworbene Resistenz gegen mindestens einen Checkpoint-inhibitorischen Antikörper, ausgewählt aus der Gruppe bestehend aus einem anti-PD-1-Antikörper, einem anti-PD-L1-Antikörper, einem anti-IDO-Antikörper, einem anti-CTLA-4-Antikörper, einem anti-Tim-3-Antikörper, einem anti-GITR-Antikörper, einem anti-VISTA-Antikörper, einem anti-BTLA-Antikörper und einem anti-OX40-Antikörper, aufweist oder (ii) wobei der Patient eine primäre oder erworbene Resistenz gegen PD-1/PD-L1-Checkpoint-Inhibition aufweist.

7. Antikörper zur Verwendung nach einem der Ansprüche 1 bis 6, wobei der Krebs ein Hirnkrebs, ein Kolorektalkrebs, ein Melanom, ein Prostatakrebs oder ein Lungenkrebs, bevorzugt ein nichtkleinzelliger Lungenkrebs (NSCLC), ist.

8. Antikörper zur Verwendung nach einem der Ansprüche 1 bis 7, wobei der Antikörper in Kombination mit Chemotherapie oder Strahlentherapie zu verwenden ist.

9. Verfahren zur Auswahl einer Therapie für einen Krebspatienten, umfassend einen Antikörper, der spezifisch die Wechselwirkung von FGL1 mit LAG3 inhibiert, wobei das Verfahren umfasst das Bestimmen des FGL1-Expressionsspiegels in einer Probe des Patienten und das Auswählen der Therapie für den Patienten, wenn der FGL1-Expressionsspiegel im Vergleich zu einem Referenzwert oder einer Referenzprobe für die Baseline-Expression von mindestens einem Subjekt, das keinen Krebs hat, oder einer nicht krebsartigen Gewebeprobe erhöht ist, wobei die nicht krebsartige Gewebeprobe bevorzugt von dem gleichen Organ wie der Krebs und bevorzugter von dem Krebspatienten stammt.

10. In-vitro-Verfahren zur Kategorisierung eines Tumors eines Patienten und/oder zur Bewertung der Empfänglichkeit eines Krebspatienten für die Behandlung mit einem Immuncheckpoint-inhibitorischen Antikörper, ausgewählt aus der Gruppe bestehend aus einem anti-PD-1-Antikörper, einem anti-PD-L1-Antikörper, einem anti-IDO-Antikörper, einem anti-CTLA-4-Antikörper, einem anti-Tim-3-Antikörper, einem anti-GITR-Antikörper, einem anti-VISTA-Antikörper, einem anti-BTLA-Antikörper, einem anti-OX40-Antikörper, einem anti-FGL1-Antikörper, einem anti-LAG3-Antikörper und bevorzugt einem Antikörper, der spezifisch die Wechselwirkung von FGL1 mit LAG3 inhibiert, umfassend
(a) Bereitstellen einer Probe von dem Patienten,
(b) Detektieren der FGL1-Expression in der Probe und
(c) Vergleichen der FGL1-Expression, die in Schritt (b) bestimmt wurde, mit einer Kontrolle, wobei die FGL1-Expression in der Probe im Vergleich zu der Kontrolle erhöht ist, wobei die Kontrolle ein Referenzwert oder eine Referenzprobe für die Baseline-Expression von mindestens einem Subjekt, das keinen Krebs hat, oder einer nicht krebsartigen Gewebeprobe ist, wobei die nicht krebsartige Gewebeprobe bevorzugt von dem gleichen Organ wie der Krebs und bevorzugter von dem Krebspatienten stammt.

11. Verfahren zur Vorhersage der Ansprechbarkeit eines menschlichen Krebspatienten, umfassend
(a) Bereitstellen einer Probe des Patienten,
(b) Bestimmen des FGL1-Expressionsspiegels in der Probe,
(c) Vergleichen der FGL1-Expression mit einer Kontrolle,
(d) Identifizieren des Patienten als wahrscheinlich ansprechend auf eine Behandlung mit einem Immuncheckpoint-inhibitorischen Antikörper, ausgewählt aus der Gruppe bestehend aus einem anti-PD-1-Antikörper, einem anti-PD-L1-Antikörper, einem anti-IDO-Antikörper, einem anti-CTLA-4-Antikörper, einem anti-Tim-3-Antikörper, einem anti-GITR-Antikörper, einem anti-VISTA-Antikörper, einem anti-BTLA-Antikörper, einem anti-OX40-Antikörper, einem anti-FGL1-Antikörper, einem anti-LAG3-Antikörper und bevorzugt einem Antikörper, der spezifisch die Wechselwirkung von FGL1 mit LAG3 inhibiert, wenn der FGL1-Expressionsspiegel im Vergleich zur Kontrolle als erhöht bestimmt worden ist, oder Identifizieren des Patienten als weniger wahrscheinlich ansprechend, wenn die FGL1-Expression im Vergleich zur Kontrolle als nicht erhöht bestimmt worden ist, wobei die Kontrolle ein Referenzwert oder eine Referenzprobe für die Baseline-Expression von mindestens einem Subjekt, das keinen Krebs hat, oder einer nicht krebsartigen Gewebeprobe ist, wobei die nicht krebsartige Gewebeprobe bevorzugt von dem gleichen Organ wie der Krebs und bevorzugter von dem Krebspatienten stammt.

12. Immuncheckpoint-inhibitorischer Antikörper, ausgewählt aus der Gruppe bestehend aus einem anti-PD-1-Antikörper, einem anti-PD-L1-Antikörper, einem anti-IDO-Antikörper, einem anti-CTLA-4-Antikörper, einem anti-Tim-3-Antikörper, einem anti-GITR-Antikörper, einem anti-VISTA-Antikörper, einem anti-BTLA-Antikörper, einem anti-OX40-Antikörper, einem anti-FGL1-Antikörper, einem anti-LAG3-Antikörper und einem Antikörper, der spezifisch die Wechselwirkung von FGL1 mit LAG3 inhibiert, zur Verwendung bei der Behandlung von Krebs in einem menschlichen Patienten, wobei der Krebspatient eine erhöhte FGL1-Expression im Vergleich zu einem Referenzwert oder einer Referenzprobe für die Baseline-Expression von mindestens einem Subjekt, das keinen Krebs hat, oder einer nicht krebsartigen Gewebeprobe aufweist, wobei die nicht krebsartige Gewebeprobe bevorzugt von dem gleichen Organ wie der Krebs und bevorzugter von dem Krebspatienten stammt.

13. Verfahren zum Screening auf einen Immuncheckpoint-inhibitorischen Antikörper oder auf ein kleines Molekül, das die Bindung von FGL1 an LAG3 inhibiert, umfassend
(a) Bereitstellen eines FGL1-Proteins, das bevorzugt mindestens die Fibrinogen-Domäne des humanen FGL1 umfasst;
(b) Bereitstellen eines LAG3-Proteins, das bevorzugt mindestens Domänen 1 und 2 des humanen LAG3 umfasst;
(c) Inkontaktbringen des FGL1-Proteins und des LAG3-Proteins in Anwesenheit eines zu screenenden Antikörpers oder eines kleinen Moleküls, z.B. aus einer Verbindungsbibliothek, z.B. im Format eines Hochdurchsatz-Screens;
(d) Bestimmen der Bindung des FGL1-Proteins an das LAG3-Protein in Anwesenheit und in Abwesenheit des Immuncheckpoint-inhibitorischen Antikörpers oder des kleinen Moleküls; und
(e) Identifizieren des zu screenenden Antikörpers als ein Antikörper, der spezifisch die Wechselwirkung des FGL1-Proteins mit dem LAG3-Protein inhibiert, wenn die Bindung des FGL1-Proteins und des LAG3-Proteins im Vergleich zur Bindung des FGL1-Proteins und des LAG3-Proteins in Abwesenheit des zu screenenden Antikörpers reduziert ist, oder Identifizieren eines kleinen Moleküls, das spezifisch die Bindung des FGL1-Proteins an das LAG3-Protein inhibiert, als ein FGL1-LAG3-Checkpoint-Inhibitor-Molekül.

14. Antikörper, der spezifisch an humanes FGL1 bindet, wobei der Antikörper die Bindung von humanem FGL1 an humanem LAG3 inhibiert.

15. Antikörper, der spezifisch an humanem LAG3 bindet, wobei der Antikörper die Bindung von humanem LAG3 an humanem FGL1 inhibiert, und wobei der Antikörper ferner die Wechselwirkung von humanem LAG3 mit humanem MHC-Klasse-Il-Molekül inhibiert.

16. Anti-FGL1-Antikörper zur Verwendung bei der Behandlung von Krebs in einem menschlichen Patienten, wobei der Krebspatient eine erhöhte FGL1-Expression im Vergleich zu einem Referenzwert oder einer Referenzprobe für die Baseline-Expression von mindestens einem Subjekt, das keinen Krebs hat, oder einer nicht krebsartigen Gewebeprobe aufweist, wobei die nicht krebsartige Gewebeprobe bevorzugt von dem gleichen Organ wie der Krebs und bevorzugter von dem Krebspatienten stammt.

## Revendications

1. Anticorps inhibant spécifiquement l'interaction de la protéine 1 de type fibrinogène (FGL1) avec la protéine du gène 3 d'activation des lymphocytes (LAG3) pour son utilisation dans le traitement d'un cancer chez un patient humain.

2. Anticorps pour son utilisation selon la revendication 1, dans lequel le patient souffrant d'un cancer présente une expression élevée de FGL1 par rapport à une valeur de référence ou à un échantillon de référence pour l'expression de ligne de base provenant d'au moins un sujet n'ayant pas de cancer ou d'un échantillon de tissu non cancéreux, dans lequel l'échantillon de tissu non cancéreux provient de préférence du même organe que le cancer et de manière davantage préférée du patient souffrant d'un cancer.

3. Anticorps pour son utilisation selon l'une quelconque des revendications 1 à 2, dans lequel ledit anticorps inhibant spécifiquement l'interaction de FGL1 avec LAG3 est un anticorps anti-FGL1 ou un anticorps anti-LAG3.

4. Anticorps se liant spécifiquement à la FGL1 humaine pour son utilisation dans le traitement d'un cancer chez un patient humain.

5. Anticorps pour son utilisation selon l'une quelconque des revendications 1 à 4, dans lequel l'anticorps doit être utilisé en combinaison avec au moins un autre anticorps inhibiteur de point de contrôle immunitaire sélectionné dans le groupe consistant en un anticorps anti-PD-1, un anticorps anti-PD-L1, un anticorps anti-IDO, un anticorps anti-CTLA-4, un anticorps anti-Tim-3, un anticorps anti-GITR, un anticorps anti-VISTA, un anticorps anti-BTLA et un anticorps anti-OX40.

6. Anticorps pour son utilisation selon l'une quelconque des revendications 1 à 5, dans lequel le patient présente (i) une résistance primaire ou acquise à au moins un anticorps inhibiteur de point de contrôle sélectionné dans le groupe consistant en un anticorps anti-PD-1, un anticorps anti-PD-L1, un anticorps anti-IDO, un anticorps anti-CTLA-4, un anticorps anti-Tim-3, un anticorps anti-GITR, un anticorps anti-VISTA, un anticorps anti-BTLA et un anticorps anti-OX40, ou (ii) dans lequel le patient présente une résistance primaire ou acquise à l'inhibition du point de contrôle PD-1/PD-L1.

7. Anticorps pour son utilisation selon l'une quelconque des revendications 1 à 6, dans lequel le cancer est un cancer du cerveau, un cancer colorectal, un mélanome, un cancer de la prostate ou un cancer du poumon, de préférence un cancer du poumon non à petites cellules (NSCLC).

8. Anticorps pour son utilisation selon l'une quelconque des revendications 1 à 7, dans lequel l'anticorps doit être utilisé en combinaison avec une chimiothérapie ou une radiothérapie.

9. Méthode de sélection d'une thérapie pour un patient souffrant d'un cancer comprenant un anticorps inhibant spécifiquement l'interaction de FGL1 avec LAG3, la méthode comprenant la détermination du niveau d'expression de FGL1 dans un échantillon provenant du patient et la sélection de ladite thérapie pour le patient quand les niveaux d'expression de FGL1 sont élevés par rapport à une valeur de référence ou à un échantillon de référence pour l'expression de ligne de base provenant d'au moins un sujet n'ayant pas de cancer ou d'un échantillon de tissu non cancéreux, dans laquelle l'échantillon de tissu non cancéreux provient de préférence du même organe que le cancer et de manière davantage préférée du patient souffrant d'un cancer.

10. Méthode in vitro de catégorisation d'une tumeur d'un patient et/ou d'évaluation de la sensibilité d'un patient souffrant d'un cancer au traitement avec un anticorps inhibiteur de point de contrôle immunitaire sélectionné dans le groupe consistant en un anticorps anti-PD-1, un anticorps anti-PD-L1, un anticorps anti-IDO, un anticorps anti-CTLA-4, un anticorps anti-Tim-3, un anticorps anti-GITR, un anticorps anti-VISTA, un anticorps anti-BTLA, un anticorps anti-OX40, un anticorps anti-FGL1, un anticorps anti-LAG3 et de préférence un anticorps qui inhibe spécifiquement l'interaction de FGL1 avec LAG3, comprenant
(a) la fourniture d'un échantillon provenant du patient,
(b) la détection de l'expression de FGL1 dans l'échantillon, et
(c) la comparaison de ladite expression de FGL1 déterminée dans l'étape (b) à un témoin, dans laquelle ladite expression de FGL1 dans ledit échantillon est élevée par rapport audit témoin, dans laquelle le témoin est une valeur de référence ou un échantillon de référence pour l'expression de ligne de base provenant d'au moins un sujet n'ayant pas de cancer ou d'un échantillon de tissu non cancéreux, dans laquelle l'échantillon de tissu non cancéreux provient de préférence du même organe que le cancer et de manière davantage préférée du patient souffrant d'un cancer.

11. Méthode de prédiction de la réceptivité d'un patient humain souffrant d'un cancer, comprenant
(a) la fourniture d'un échantillon provenant du patient,
(b) la détermination du niveau d'expression de FGL1 dans ledit échantillon,
(c) la comparaison de l'expression de FGL1 à un témoin,
(d) l'identification que le patient est susceptible d'être réceptif au traitement avec un anticorps inhibiteur de point de contrôle immunitaire sélectionné dans le groupe consistant en un anticorps anti-PD-1, un anticorps anti-PD-L1, un anticorps anti-IDO, un anti-CTLA-4, un anticorps anti-Tim-3, un anticorps anti-GITR, un anticorps anti-VISTA, un anticorps anti-BTLA, un anticorps anti-OX40, un anticorps anti-FGL1, un anticorps anti-LAG3 et de préférence un anticorps qui inhibe spécifiquement l'interaction de FGL1 avec LAG3, quand il a été déterminé que le niveau d'expression de FGL1 est élevé par rapport au témoin, ou l'identification que le patient est moins susceptible d'être réceptif quand il a été déterminé que l'expression de FGL1 n'est pas élevée par rapport au témoin, dans laquelle le témoin est une valeur de référence ou un échantillon de référence pour l'expression de ligne de base provenant d'au moins un sujet n'ayant pas de cancer ou d'un échantillon de tissu non cancéreux, dans laquelle l'échantillon de tissu non cancéreux provient de préférence du même organe que le cancer et de manière davantage préférée du patient souffrant d'un cancer.

12. Anticorps inhibiteur de point de contrôle immunitaire sélectionné dans le groupe consistant en un anticorps anti-PD-1, un anticorps anti-PD-L1, un anticorps anti-IDO, un anticorps anti-CTLA-4, un anticorps anti-Tim-3, un anticorps anti-GITR, un anticorps anti-VISTA, un anticorps anti-BTLA, un anticorps anti-OX40, un anticorps anti-FGL1, un anticorps anti-LAG3 et un anticorps qui inhibe spécifiquement l'interaction de FGL1 avec LAG3, pour une utilisation dans le traitement d'un cancer chez un patient humain, dans lequel le patient souffrant d'un cancer présente une expression élevée de FGL1 par rapport à une valeur de référence ou à un échantillon de référence pour l'expression de ligne de base provenant d'au moins un sujet n'ayant pas de cancer ou d'un échantillon de tissu non cancéreux, dans lequel l'échantillon de tissu non cancéreux provient de préférence du même organe que le cancer et de manière davantage préférée du patient souffrant d'un cancer.

13. Méthode de criblage d'un anticorps inhibiteur de point de contrôle immunitaire ou d'une petite molécule qui inhibe la liaison de FGL1 à LAG3, comprenant
(a) la fourniture d'une protéine FGL1, comprenant de préférence au moins le domaine fibrinogène de la FGL1 humaine ;
(b) la fourniture d'une protéine LAG3, comprenant de préférence au moins les domaines 1 et 2 de la LAG3 humaine ;
(c) la mise en contact de ladite protéine FGL1 et de ladite protéine LAG3 en présence d'un anticorps à cribler ou d'une petite molécule, par exemple provenant d'une banque de composés, par exemple sous la forme d'un criblage à haut débit ;
(d) la détermination de la liaison de ladite protéine FGL1 à ladite protéine LAG3 en présence et en l'absence dudit anticorps inhibiteur de point de contrôle immunitaire ou de ladite petite molécule ; et
(e) l'identification que l'anticorps à cribler est un anticorps qui inhibe spécifiquement l'interaction de ladite protéine FGL1 avec ladite protéine LAG3 si la liaison de ladite protéine FGL1 et de ladite protéine LAG3 est réduite par rapport à la liaison de ladite protéine FGL1 et de ladite protéine LAG3 en l'absence dudit anticorps à cribler ou l'identification qu'une petite molécule qui inhibe spécifiquement la liaison de ladite protéine FGL1 à ladite protéine LAG3 est une molécule inhibitrice du point de contrôle FGL1-LAG3.

14. Anticorps qui se lie spécifiquement à la FGL1 humaine, dans lequel l'anticorps inhibe la liaison de la FGL1 humaine à la LAG3 humaine.

15. Anticorps qui se lie spécifiquement à la LAG3 humaine, dans lequel l'anticorps inhibe la liaison de la LAG3 humaine à la FGL1 humaine, et dans lequel l'anticorps inhibe en outre l'interaction de la LAG3 humaine avec la molécule CMH de classe II humaine.

16. Anticorps anti-FGL1 pour son utilisation dans le traitement d'un cancer chez un patient humain, dans lequel le patient souffrant d'un cancer présente une expression élevée de FGL1 par rapport à une valeur de référence ou à un échantillon de référence pour l'expression de ligne de base provenant d'au moins un sujet n'ayant pas de cancer ou d'un échantillon de tissu non cancéreux, dans lequel l'échantillon de tissu non cancéreux provient de préférence du même organe que le cancer et de manière davantage préférée du patient souffrant d'un cancer.
